(19) Europäisches Patentamt
European Patent Office
Office européen des brevets

(11) Publication number: 0 326 640 B1

# (12) EUROPEAN PATENT SPECIFICATION

(45) Date of publication of patent specification: 28.04.93

(51) Int. Cl.[5]: C07D 487/04, A61K 31/40, //(C07D487/04,209:00,205:00)

(21) Application number: 88112142.0

(22) Date of filing: 27.07.88

## (54) (1R,5S,6S)-2-substituted thio-6 (R)-1-hydroxyethyl -1-methyl-carbapenem - carboxylic acid derivatives.

(30) Priority: 07.12.87 US 129555

(43) Date of publication of application:
09.08.89 Bulletin 89/32

(45) Publication of the grant of the patent:
28.04.93 Bulletin 93/17

(84) Designated Contracting States:
AT BE CH DE ES FR GB GR IT LI LU NL SE

(56) References cited:
EP-A- 0 006 639
EP-A- 0 162 242
EP-A- 0 170 073
FR-A- 2 533 568

(73) Proprietor: LEDERLE (JAPAN) LTD.
Hattori Bldg., 5th Floor 10-3 Kyobashi
1-chome
Chuo-ku Tokyo(JP)

(72) Inventor: Aoyagi, Sakae
3-6-1004, Tate 2-chome
Shiki-shi Saitama-ken(JP)
Inventor: Kobayashi, Shigeaki
22-27, Fujimi 2-chome Tsurugashima-cho
Iruma-gun Saitama-ken(JP)

(74) Representative: Kraus, Walter, Dr. et al
Patentanwälte Kraus, Weisert & Partner
Thomas-Wimmer-Ring 15
W-8000 München 22 (DE)

EP 0 326 640 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a carbapenem antibiotic and, more particularly, to crystalline 1 - methyl-carbapenem having a methyl group introduced at the 1-position of the carbapenem skeleton and a (methyl-1,2,3-thiadiazolium-4-yl)methylthio group as a quaternary ammonium functional group at the 2-position thereof, to an antibacterial composition containing the same as an active ingredient, and to a process of preparing the same.

Heretofore, as various antibacterial substances, there have been proposed many carbapenem antibiotic substances having, as a basic skeleton, carba-2-penem-3-carboxylic acid represented by the following formula (A):

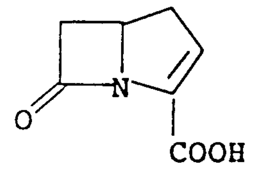

(A)

For example, an initial generation of carbapenem antibiotics is a naturally occurring carbapenem compound such as thienamycin represented by the formula (B):

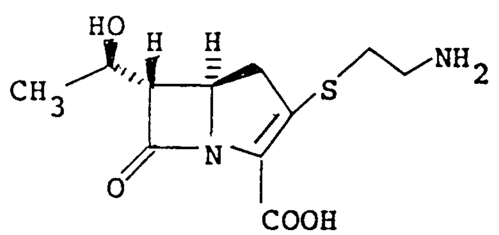

(B)

The thienamycin may be obtained from a fermentation broth of Streptomyces cattleya and has a broad range of antibacterial spectra against Gram-positive and Gram-negative bacteria. It has been expected therefore to be developed as a highly useful compound, but its poor chemical stability has precluded its commercialization.

With the foregoing background, many researchers have attempted to develop a carbapenem compound having antibacterial activities as high as thienamycin and ensuring more chemical stability. As a result, there has been developed imipenem (INN) represented by the following formula (C):

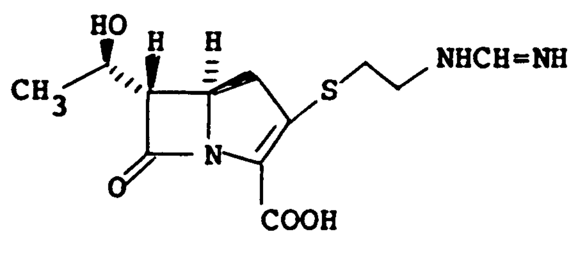

(C)

This compound is a practically available antibacterial agent and may be obtained by converting an amino group as a side chain at the 2-position to a formimidoyl group.

The imipenem of formula (C) exhibits antibacterial activities higher than those of the thienamycin and ensures some degree of chemical stability; however, it presents the disadvantage that it is decomposd within a short period of time by kidney dehydropeptidase (DHP) in the living body. For this reason, it cannot be administered singly, and must be used in combination with a DHP inhibitor in order to control its decomposition leading to inactivation. Its formulation for clinical administration is a combination with cilastatin (INN) that is a DHP inhibitor.

An antibacterial agent preferred for practical clinical use, however, is one that alone can demonstrate antibacterial activity. Furthermore, the DHP inhibitor to be combined with the antibiotic could exert undesirable actions on tissues of the living body. For these reasons, the combined use should be avoided

wherever possible. Thus there has been a growing demand for a carbapenem compound having sufficiently high degrees of both antibacterial activity and resistance to DHP.

Recently, there were proposed some carbapenem compounds of the type that could achieve the above objectives. Such carbapenem compounds are 1-methyl-carbapenem compounds in which a methyl group is introduced at the 1-position of the carbapenem skeleton. Most recently, another type of carbapenem compounds was proposed which has a heterocycloalkylthio group at the 2-position of the carbapenem skeleton. For example, European Patent Publication No. 170,073 (Japanese Laid-Open Patent Publication No. 83,183/1986) to Merck discloses 1-methylcarbapenem compounds having at the 2-position an alkylated mono- or bi-cyclic quaternary heteroaryl alkylthio substituent, represented by the formula (D):

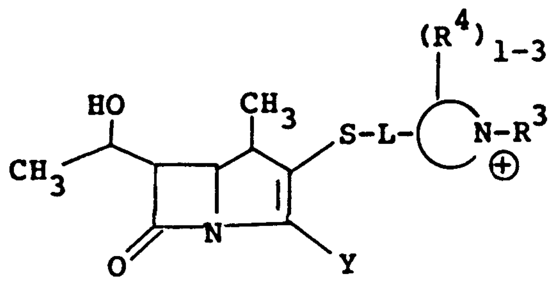

(D)

It is reported that these compounds have superior antibacterial activities as well as a remarkably improved resistance to decomposition by DHP leading to inactivation so that they demonstrate highly useful effects.

It should be noted here that the Japanese Patent Laid-Open Publication No. 83,183/1986 discloses merely a general concept of 1$\beta$-methyl-carbapenem compounds and a very limited number of specific working examples. It also discloses in generic terms that they are superior in antibacterial activities; however, it does not specifically describe any antibacterial data whatsoever. Furthermore, the Japanese patent document merely enumerates more than about 470 compounds by their names only; but it only contains 10 compounds in amorphous form after lyophilization which are actually supported by working examples. No specific compounds according to the present invention are disclosed therein and the prior patent application quoted above does not hint anything about such compounds having superior pharmacological characteristics as demonstrated and claimed by the present invention.

Furthermore, FR-A-2 533 568 corresponding to U.S. Patent 4,644,061 assigned to Bristol Myers discloses carbapenem compounds with a quaternary heteroalkylthio substituent at the 2-position of the carbapenem skeleton, as represented by the formula (E):

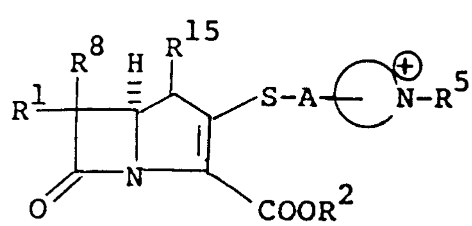

(E)

wherein $R^1$ is a hydroxyethyl group; $R^8$ is a hydrogen atom; and $R^{15}$ is a hydrogen atom or a methyl group.

The specification of this U. S. Patent describes a compound in which the group

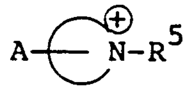

is a (2-methyl-1,2,3-thiadiazolium-4-yl)methyl group of the formula

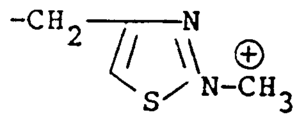

(for example, Example 16). This compound is a lyophilized product in an amorphous form.

In actually commercializing carbapenem-series compounds, they are desirably in a crystalline form in view of their chemical stability. In particular, an amorphous solid has insufficient stability, and when stored for a long period of time under ordinary conditions, it discolors and decreases in activity. If a carbapenem-series compound is to be marketed as an amorphous solid, a complex purification step is required to produce it as a substantially pure form, and various problems exist in its commercialization.

Thus, in spite of the great importance of obtaining crystalline carbapenem compounds, no detailed investigation has been made on their crystals.

The present invention provides a crystalline carbapenem compound having high antibacterial activities, a strong action of inhibiting β-lactamase as well as improved resistance to kidney dehydropeptidase. More specifically, the present invention provides the crystalline carbapenem compound substituted by a methyl group at the 1-position in the β-configuration, in which particularly a (methyl-1,2,3-thiadiazolium-4-yl)-methylthio group is introduced at the 2-position.

Specifically, the present invention provides crystalline (1R,5S,6S)-2-[(2-methyl-1,2,3-thiadiazolium-4-yl)-methyl]thio-6-[(R)-1-hydroxyethyl]-1-methyl-carbapenem-3-carboxylate represented by the following formula:

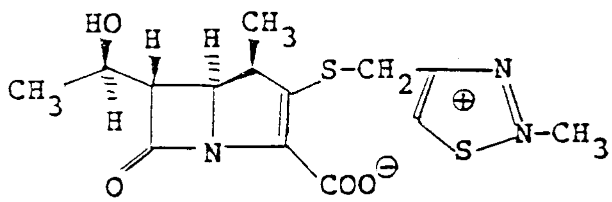

(I-1)

The present invention further provides an antibacterial composition containing the crystalline carbapenem compound represented by formula (I-1) as an active ingredient.

The compound of formula (I-1) corresponds to a carbapenem compound in which the 1-position is in the beta-configuration and substituted by methyl and which has not previously been studied in detail. The present inventors found that the compound of formula (I-1) is unique in that a (methyl-substituted-1,2,3-thiadiazolium-4-yl)methylthio group is introduced as a 2-position side chain and this side chain forms an intramolecular salt with the carboxylate at the 3-position; and also found that this compound has excellent antibacterial activity.

The present invention is based on the discovery that the above unique carbapenem compound of formula (I-1) can be obtained as stable crystals, and can be easily purified as a result of crystallization. The compound is markedly characterized by the fact that as a result of crystallization, its antibacterial activity and resistance to DHP are uniquely excellent.

Observation under a polarizing microscope and powder X-ray diffractometry can lead to the determination that the carbapenem compound of formula (I-1) provided by this invention is crystalline. This is particularly based on the fact that the powder X-ray diffraction pattern of the above compound has characteristic peaks at a spacing (d) of 6.9, 5.3, 4.6, 4.2, 3.9, 3.3, 3.0, 2.5 and 2.4 Å.

In accordance with the present invention, the carbapenem compound represented by formula (I-1) may be prepared basically by a process to be described below.

Briefly stated, the carbapenem of formula (I-1) may be prepared by reacting a compound represented by formula (II):

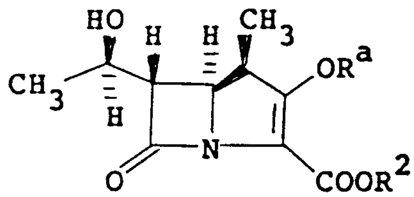

(II)

wherein $R^2$ is a carboxyl protecting group, and $R^a$ is an acyl group, with a mercapto reagent represented by formula (III):

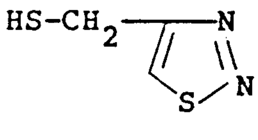

(III)

to give a compound represented by formula (IV):

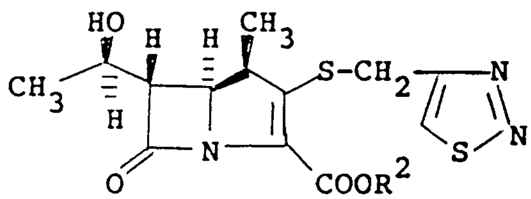

(IV)

wherein $R^2$ has the same meaning as above, and then subjecting the compound of formula (IV) first to removal of the carboxyl protecting group $R^2$ and then to quaternization by dimethyl sulfate or methyl triflate or, alternatively, first to quaternization with the dimethyl sulfate or the methyl triflate and then to removal of the carboxyl protecting group $R^2$, to give the carbapenem compound represented by formula (I-1).

Furthermore, the carbapenem compound represented by formula (I-1) may be prepared by reacting the compound represented by formula (II) with a mercapto reagent represented by formula (III-1):

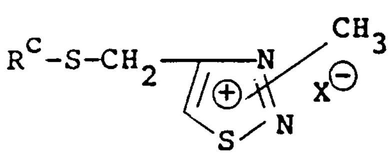

(III-1)

wherein $R^c$ is a mercapto protecting group and $X^{\ominus}$ is a residue of a quaternary ammonium salt, to give a compound represented by formula (IV-I):

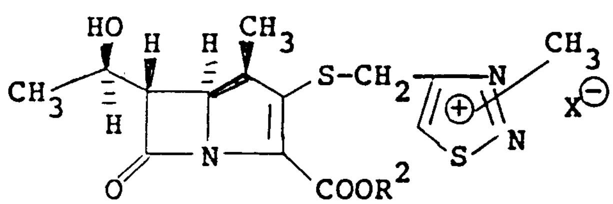

(IV-1)

wherein $R^2$ and $X^{\ominus}$ have the same meanings as above, and subjecting the compound represented by formula (IV-1) to removal of the carboxyl protecting group $R^2$ to give the carbapenem compound represented by formula (I-1).

More specifically, the carbapenem compound represented by formula (I-1) may be prepared in such a manner as will be described in detail below.

The carbapenem compound represented by formula (II) to be employed as a starting compound in the process described above is known per se and may be prepared in such a manner as disclosed, for example, in U. S. Patent 4,312,871 (Japanese Patent Laid-Open Publication No. 123,985/1981) or, more preferably, in accordance with the spatially selective method as indicated in Reaction Scheme A below and proposed by the present inventors (as disclosed, for example, in Japanese Patent Application No. 315,444/1986).

## REACTION SCHEME A

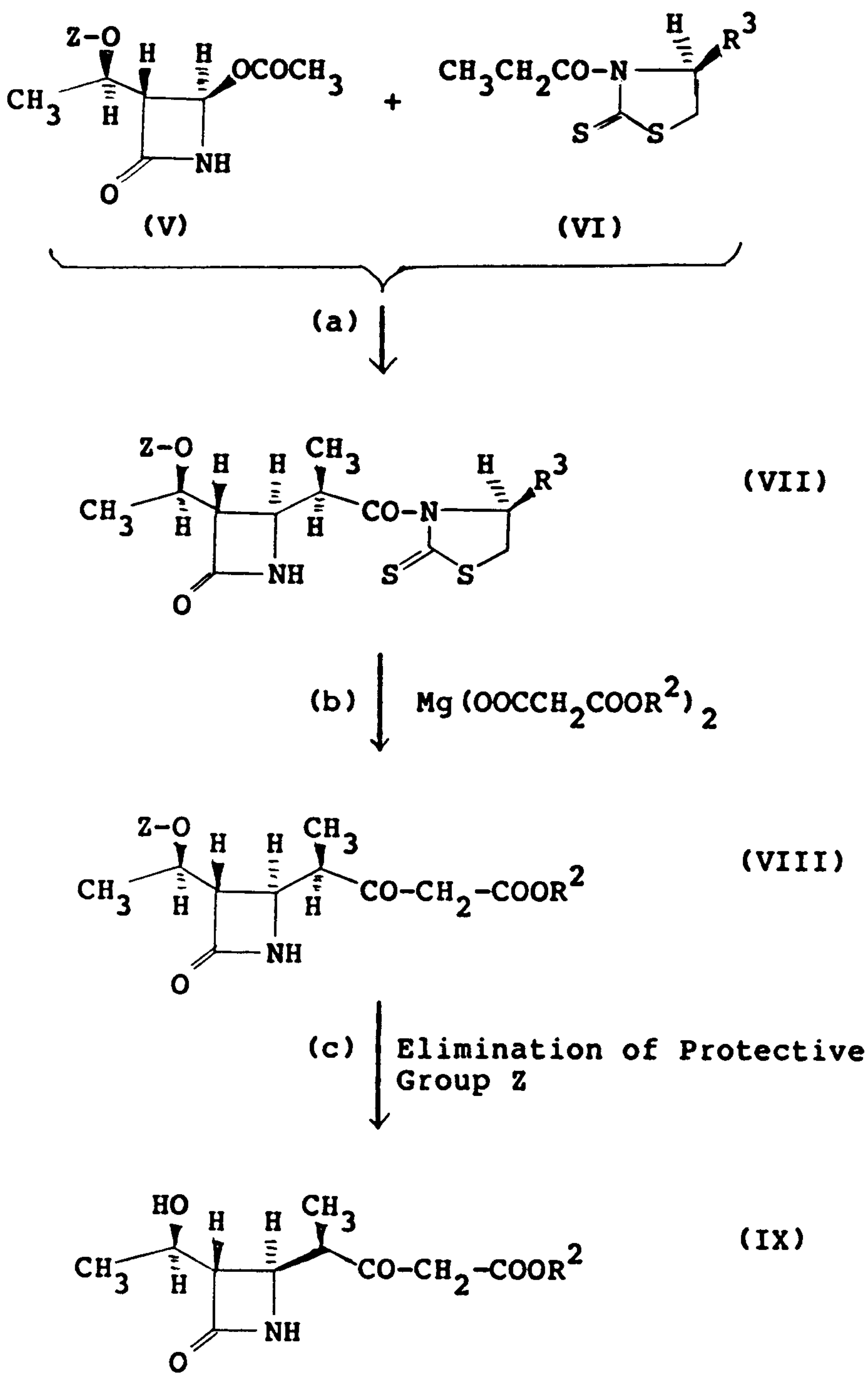

(V) (VI) (VII) (VIII) (IX)

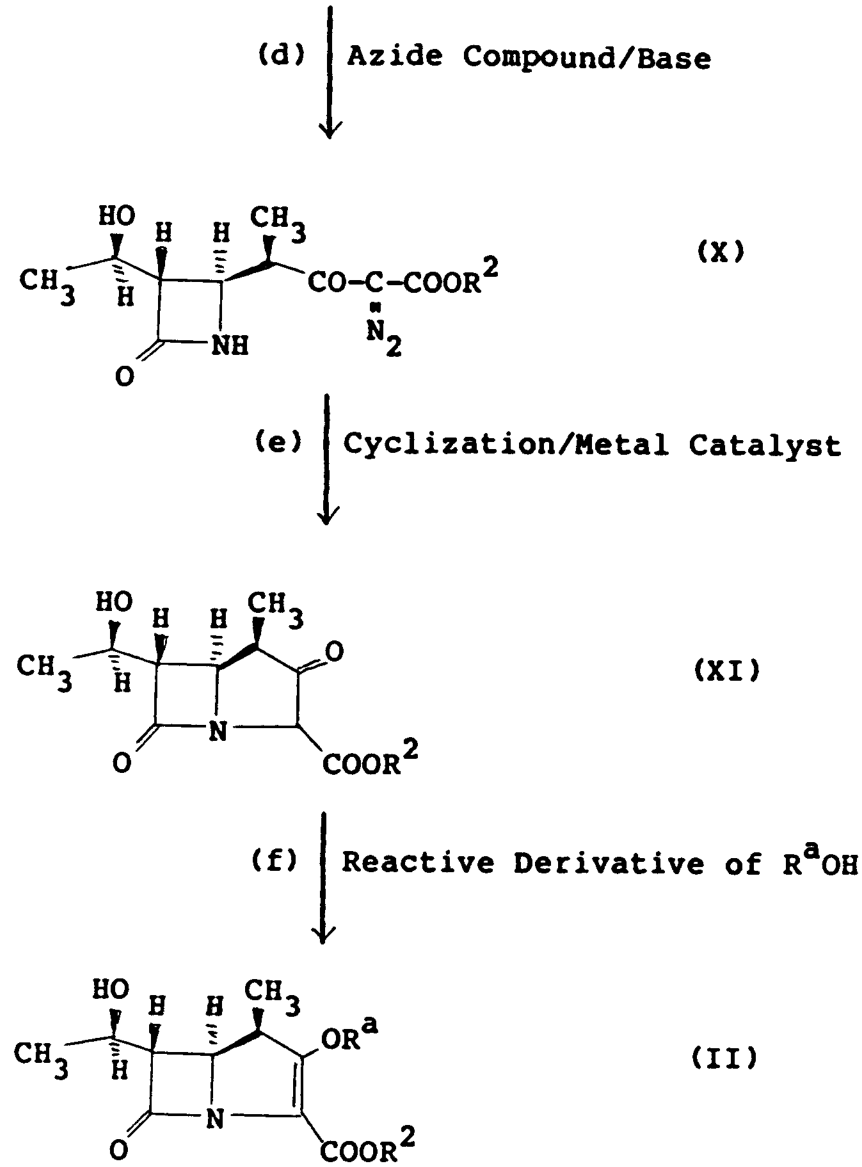


(X)

(XI)

(II)

wherein $R^3$ is a hydrogen atom or a lower alkyl group; Z is tertiary-butyldimethylsilyl group; and $R^2$ and $R^a$ have the same meanings as above.

In the specification of the present application, the term "lower" qualifying a group or a compound means that the group or compound so qualified has from 1 to 7, preferably from 1 to 4, carbon atoms.

The term "lower alkyl" referred to herein stands for a straight-chained or branched-chain hydrocarbon group having preferably from 1 to 6 carbon atoms and may include, for example, methyl, ethyl, n-propyl, isopropyl, n-butyl, isobutyl, sec-butyl, tert.-butyl, n-pentyl, isopentyl, n-hexyl or isohexyl.

The term "carboxyl protecting group" referred to herein stands for any group capable of protecting the carboxyl group of the compound involved without adversely affecting any other substituents and the reactions that follow and may include, for example, an ester residue such as a lower alkyl ester residue including, for example, methyl ester, ethyl ester, n-propyl ester, isopropyl ester, n-, iso-, sec- or tert.-butyl ester or n-hexyl ester; an aralkyl ester residue including, for example, benzyl ester, p-nitrobenzyl ester, o-nitrobenzyl ester or p-methoxybenzyl ester; and a lower aliphatic acyloxymethyl ester residue including, for example, acetoxymethyl ester, propionyloxymethyl ester, n- or iso-butyryloxymethyl ester or pivaloyloxymethyl ester.

The term "acyl group" referred to herein stands for, in a narrower sense, a moiety obtainable by removing the hydroxyl group from the carboxyl group of an organic carboxylic acid as well as, in a broader sense, any acyl group derived from an organic sulfonic acid or an organic phosphoric acid. Such an acyl group may include, for example, a lower alkanoyl group such as acetyl, propionyl or butyryl, a (halo)lower

alkyl sulfonyl group such as methanesulfonyl or trifluoromethanesulfonyl; a substituted or unsubstituted arylsulfonyl group such as benzenesulfonyl, p-nitrobenzenesulfonyl, p-bromobenzenesulfonyl, toluenesulfonyl or 2,4,6-triisopropylbenzenesulfonyl; and diphenylphosphoryl.

The term "mercapto protecting group" referred to herein stands for any group capable of protecting the mercapto group of the compound involved without adversely affecting the compounds and the process according to the present invention and may include, for example, a lower alkanoyl group such as acetyl or propionyl; a silyl group such as tert.-butyldimethylsilyl, tert.-butyldiphenylsilyl or (2-phenyl-2-propyl)-dimethylsilyl.

Each of the steps of the Reaction Scheme A above for preparing the compounds represented by formula (II) in a highly spatial selectivity will be described below more in detail.

The step (a) involves the reaction of the N-propionyl-1,3-thiazoline-2-thione derivative of formula (VI) with a tin(II) triflate in the presence of a base to give an enolate and then the reaction of the resulting enolate with the compound of formula (V) to give the azetidin-2-one derivative of formula (VII).

The enolization reaction of the N-propionyl-1,3-thiazoline-2-thione derivative of formula (VI) with the tin-(II) triflate may be carried out usually in an inert solvent such as an ether, i.e., diethyl ether or tetrahydrofuran; a hydrocarbon, i.e., toluene or cyclohexane; a halogenated hydrocarbon, i.e., dichloromethane or chloroform. Preferably tetrahydrofuran can be used.

The reaction temperature is not limited to a particular range of temperatures and may vary in a wide range with starting materials to be used. Usually the reaction temperature may be in a range of relatively low temperatures as low as from approximately -100 ° C to about room temperature, preferably from approximately from -78 ° C to approximately 0 ° C.

The quantity of the tin(II) triflate with respect to the compound of formula (VI) is not critical and may range usually from approximately 1 mole to approximately 2 moles, preferably from 1 to 1.5 moles, per mole of the compound of formula (VI).

The enolization reaction above is carried out usually in the presence of the base including, for example, a tertiary amine such as triethylamine, diisopropylethyl amine, 1,4-diazabicyclo[2.2.2]octane, N-methylmorpholine, N-ethylpiperidine or pyridine. N-Ethylpiperidine is employed advantageously. The base may be used at a rate ranging generally from approximately 1.0 to approximately 3 molar equivalents, preferably from 1.0 to 2 molar equivalents, per mole of the compound of formula (VI).

The enolization reaction as described above may be completed generally in approximately 5 minutes to approximately 4 hours, thus leading to the formation of the enolate.

After completion of the enolization reaction, the resulting enolate may be used as it is for further reaction with the compound of formula (V).

The resulting enolate is then subjected to the alkylation reaction with the compound of formula (V). The alkylation reaction may be conducted at temperatures in the range generally from approximately -100 ° C to about room temperature, preferably from approximately -78 ° C to approximately 10 ° C. The quantity of the compound of formula (V) is not critical and may vary conveniently in a range generally from approximately 0.5 mole to approximately 5 moles, preferably from 0.5 to 2 moles, per mole of the compound of formula (VI) used for the enolization.

The alkylation reaction may be carried out under such conditions as described above generally for approximately 5 minutes to approximately 5 hours, preferably for 5 minutes to approximately 2 hours.

The enolization and alkylation reactions may be carried out preferably in an inert atmosphere such as an atmosphere of nitrogen gas or argon gas.

The reaction product obtained by the above reaction is then treated with water. For instance, after completion of the reaction, a phoshate buffer with approximately pH 7 is added, and a mixture is then stirred to be followed by filtration of undissolved materials. After filtration, the compound of formula (VII) is separated and purified in conventional manner, such as by means of extraction, recrystallization and chromatography.

The step (b) is a step by which the compound of formula (VIII) may be prepared by reacting the azetidin-2-one derivative of formula (VII) obtained by the step (a) above with a magnesium malonate represented by the general formula: $(R^2OOCCH_2CO_2)_2Mg$ in the presence of imidazole.

The reaction is carried out preferably in an inert organic solvent such as an ether solvent, i.e., ether, tetrahydrofuran or dioxane; a hydrocarbon solvent, i.e., toluene, xylene or cyclohexane; a halogenated hydrocarbon solvent, i.e., dichloromethane or chloroform; and acetonitrile. Particularly acetonitrile may be employed conveniently.

The reaction temperature is not limited strictly to a particular range and may vary in a wide range with starting materials to be used or the like. They may range generally from approximately 0 ° C to approximately 100 ° C, preferably around room temperature.

The quantity of the magnesium malonate with respect to the compound of formula (VII) may be about an equimolar amount, and the reaction may be completed in approximately 50 hours, preferably in approximately 20 hours.

The magnesium malonate to be used may include, for example, p-nitrobenzylmagnesium malonate, benzylmagnesium malonate or methylmagnesium malonate. Among them, p-nitrobenzylmagnesium malonate is preferably used.

The step (c) is a step to eliminate a hydroxyl protecting group Z from the compound of formula (VIII) obtained by the step (b) above. The tertiary-butyldimethylsilyl group as the hydroxyl protecting group Z may be eliminated by subjecting the compound of formula (VIII) to acidic hydrolysis in a solvent such as methanol, ethanol, tetrahydrofuran or dioxane in the presence of an acid such as a mineral acid, i.e., hydrochloric acid, sulfuric acid or an organic acid, i.e., acetic acid at temperatures ranging from 0 ° C to 100 ° C for reaction periods ranging from 0.5 to 18 hours.

The above step may yield the compound represented by formula (IX) in a quantitative amount.

The step (d) is a step by which the diazo compound of formula (X) may be prepared by treating the compound of formula (IX) obtainable by the above step (c) with an azide compound in the presence of a base in such an inert organic solvent as have been enumerated for the step (d) above.

The azide compound to be used in the step (d) may include, for example, p-carboxylbenzenesulfonyl azide, toluenesulfony azide, methanesulfonyl azide or dodecylbenzenesulfonyl azide. The base to be used therein may include, for example, triethylamine, pyridine or diethylamine.

The reaction may be carried out, for instance, by adding p-toluenesulfonyl azide to acetonitrile preferably in the presence of triethylamine at 0 ° C to 100 ° C, preferably at room temperature for 1 to 50 hours. This reaction produces the diazo compound represented by formula (X) in a high yield.

The step (e) is a step by which the diazo compound of the formula (X) obtainable by the step (d) above may be cyclized to give the compound of formula (XI). This step may be carried out preferably in an inert solvent such as benzene, toluene, tetrahydrofuran, cyclohexane, ethyl acetate or dichloromethane, preferably in toluene, at temperatures ranging from 25 ° C to 110 ° C for 1 to 5 hours in the presence of a metal catalyst such as a metal carboxylate compound including, for example, bis(acetylacetonate)Cu(II), $CuSO_4$, copper powder, $Rh_2(OCOCH_3)_4$, rhodium octanoate or $Pb(OCOCH_3)_4$. As an alternative procedure, the above cyclization step may be carried out by subjecting the compound of formula (X) to irradiation from a light source through a Pyrex filter (its wavelength being larger than 300 nm) in a solvent such as benzene or diethyl ether at 0 ° C to 250 ° C for 0.5 to 2 hours.

The step (f) produces the compond of formula (II) by reacting the compound of formula (XI) obtainable by the step (e) with a reactive derivative of an acid represented by the formula: $R^aOH$. The reactive acid derivative may include, for example, an acid anhydride such as acetic acid anhydride, methanesulfonic acid anhydride, p-toluenesulfonic acid anhydride, p-nitrobenzenesulfonic acid anhydride, 2,4,6-triisopropylbenzenesulfonic acid anhydride or trifluoromethanesulfonic acid anhydride or an acid halide such as an acid chloride, i.e., acetyl chloride, propionyl chloride, diphenylphosphoric chloride, toluenesulfonyl chloride or p-bromobenzenesulfonyl chloride. Diphenylphosphoric chloride ($R^a$ = diphenylphosphoryl group) is particularly preferred.

The reaction of the compound of formula (XI) with the reactive acid derivative may be carried out, for example, in a manner similar to a conventional acylation reaction in an inert solvent such as methylene chloride, acetonitrile or dimethylformamide, conveniently in the presence of a base such as diisopropylethyl amine, triethylamine or 4-dimethylaminopyridine at temperatures ranging from -20 ° C to 40 ° C for approximately 30 minutes to approximately 24 hours.

The reaction consisting of a series of the steps as have been described above provides the compound represented by formula (II) with a highly spatial selectivity and with such a spatial arrangement that the methyl group at the 1-position of the carbapenem skeleton is arranged in the R configuration, the substituent at the 5-position thereof is in the R configuration, and the substituent and the hydroxy group each at the 6-position thereof are in the S and R configurations, respectively.

The compound represented by formula (II) is then reacted with a mercapto reagent represented by formula (III) or (III-1) to give the compound represented by formula (IV) or (IV-1) respectively.

The reaction of the compound of formula (II) with the mercapto reaction of formula (III) or (III-1) may be carried out, for instance, by reacting the compound of formula (II) with the mercapto reagent of formula (III) or (III-1) in an excess amount ranging from about an equimolar amount to approximately 1.5 molar amount in an appropriate solvent such as tetrahydrofuran, dichloromethane, dioxane, dimethylformamide, dimethylsulfoxide; acetonitrile or hexamethylene phosphoramide, preferably in the presence of a base such as sodium hydrogen carbonate, potassium carbonate, triethylamine or diisopropylethyl amine at a temperature range from approximately -40 ° C to approximately 25 ° C for approximately 30 minutes to approximately 24

hours.

The reaction described above provides the carbapenem compound represented by formula (IV) or (IV-1) in which the carboxyl group at the 3-position thereof is protected by the carboxyl protecting group $R^2$. The removal of the protecting group $R^2$ may be made by a reaction known per se for removing a protective group, such as solvolysis or hydrogenolysis. In a typical reaction, the compound represented by formula (IV) or (IV-1) may be treated, for instance, in a mixture of solvents such as tetrahydrofuran-water, tetrahydrofuran-ethanol-water, dioxane-water, dioxane-ethanol-water or n-butanol-water containing morpholino-propane sulfonic acid-sodium hydroxide buffer solution (pH 7), a phosphate buffer solution (pH 7), dipotassium phosphate or sodium bicarbonate, using hydrogen under 1 to 4 atmospheric pressures, in the presence of a catalyst for hydrogenation such as platinum oxide, palladium-activated carbon or palladium hydroxide-activated carbon at temperatures ranging from approximately 0 °C to approximately 50 °C for approximately 0.25 to approximately 4 hours.

In the compound of formula (IV), the carboxyl group in a free state (where $R^2$ is hydrogen atom) is then subjected to quaternization to provide the carbapenem compound of formula (I-1) according to the present invention. The quaternization reaction may be preferably conducted usually by dissolving the compound of formula (IV), for instance, in a phosphate buffer solution (pH 7) and then causing a dimethyl sulfate to act on the resultant solution or methyl triflate to act on the resultant solution in an appropriate solvent such as dioxane, acetonitrile or tetrahydrofuran. The resulting compound can be converted into the objective carbapenem compound represented by formula (I-1) as an intramolecular amphoteric compound, using an appropriate ion exchange resin such as, preferably, Dowex 50W-X4 type ion exchange resin and lyophilization.

In the above steps, the removal reaction of the carboxyl protecting group Z and the quaternization reaction may be carried out in the reverse order from the above order. In other words, the removal reaction may be conducted after the quaternization step. Usually the reaction in this order is preferred.

On the other hand, the compound of formula (IV-1) obtained by the reaction of the compound of formula (II) with the mercapto reagent of formula (III-1) can be converted into the objective carbapenem compound of formula (I-1) by the same removal reaction of the carboxyl protecting group $R^2$ as have been described above.

The amorphous (1R,5S,6S)-2-[(2-methyl-1,2,3-thiadiazolium-4-yl)methyl]thio-6-[(R)-1-hydroxyethyl]-1-methyl-carbapenem-3-carboxylate of formula (I-1) obtained as above can be converted into the desired crystalline compound in accordance with this invention. The crystallization can be carried out specifically by the procedure to be described.

The compound of formula (I-1) in the lyophilized state (amorphous) produced by the process described above is treated with a suitable combination of ion-exchange resins such as Dowex 50W-X4, Amberlite IR-120 and Diaion HP-40 to increase its purity, preferably to at least 97%, especially preferably to at least 99%. The purified amorphous compound of formula (I-1) is crystallized by selecting water as a crystallization solvent. More specifically, the amorphous compound of formula (I-1) is completely dissolved in water, optionally with stirring, so as to provide a solution having a concentration of at least 65% (w/w), preferably at least 67% (w/w), and crystals are precipitated from the aqueous solution. This can be achieved by stirring the aqueous solution obtained as above. Conveniently, this stirring is carried out generally at a temperature of about 0 °C to about 40 °C, preferably at room temperature for a period of time sufficient for the crystals in accordance with this invention to precipitate from the stirred solution.

It has been found in accordance with this invention that in performing the crystallization in accordance with this invention from the aqueous solution of the amorphous compound of formula (I-1), it is important to use the amorphous compound of a high purity as the starting material and water as the crystallization solvent and to precipitate the crystals of the compound from the resulting high-concentration solution. Investigations of the present inventors have shown that when the amorphous compound of formula (I-1) having a purity of less than 97% is used as the starting material and solvents usually used in the crystallization of organic compounds such as lower alcohols (e.g., methanol, ethanol, isopropanol or n-propanol), tetrahydrofuran, acetone or ethyl acetate, either singly or in combination with water, are used as the crystallization solvent, it is difficult to form the desired crystalline compound of formula (I-1) contemplated by this invention. If water alone is used as the crystallization solvent but the amorphous compound of formula (I-1) having a purity of less than 97% is used as the starting material, the crystallization is also difficult.

However, if crystals of the compound of formula (I-1) once obtained are used as seed crystals, it is not always necessary to use the amorphous compound of formula (I-1) having a high purity as the starting material and/or water as the sole crystallization solvent. In this case, the amorphous compound of formula (I-1) having a purity of about 97% is used as the starting material and dissolved in water to a certain

concentration of, for example, at least about 55% (w/w), and a small amount of a seed of the crystalline compound of formula (I-1) is added to the solution. A water-miscible organic solvent such as ethanol is further added, and the mixture is stirred to give the desired crystalline compound of formula (I-1).

The crystals precipitated as above may be directly filtered and dried in a customary manner to give the crystals of the invention when only water is used as the crystallization solvent and the amorphous compound of formula (I-1) used in the crystallization has a sufficiently high purity. Since in the crystallization operation in accordance with this invention, undesirable impurities move to the mother liquor, the desired crystals having a sufficient purity can be obtained also by filtering the solution containing the precipitated crystals and washing the resulting crystals with a small amount of an organic solvent or a mixture of water and an organic solvent. Examples of such an organic solvent are ethanol, isopropanol, n-propanol and acetone. Ethanol is most preferred.

As shown in Examples to be described, observation under a polarizing microscope and powder X-ray diffractometry show that the resulting compound of formula (I-1) produced by the present invention, namely (1R,5S,6S)-2-[methyl-1,2,3-thiadiazolium-4-yl)methyl]-thio-6-[(R)-1-hydroxyethyl]-1-methyl-carbapenem-3-carboxylate, is crystalline. The stability of the resulting crystal compound is much higher than the corresponding amorphous compound and its antimicrobial activity is strong. Hence, the crystalline compound of formula (I-1) is very useful as a therapeutic antibacterial agent.

The desired crystalline compound of formula (I-1) in accordance with the present invention is a novel crystalline compound that is not disclosed specifically in the above-mentioned publication and that is extremely stable against dehydropeptidase (DHP) known as a kidney enzyme and has superior antibacterial activities. The remarkably high antibacterial activities and stability against the kidney DHP of the crystalline compound of formula (I-1) according to the present invention have been determined by biological tests described below.

I. Antibacterial Tests

Test Procedures:

The antibacterial activities were tested by an agar plate dilution method in accordance with the standard method of The Japanese Chemotherapy Society [Chemotherapy, Vol. 29, 76-79 (1981)].

A Mueller-Hinton (MH) agar liquid medium of a test microorganism was cultured overnight at 37° C and the resultant culture medium was diluted with a buffered saline gelatin (BSG) solution to contain approximately $10^6$ cells of the test microorganisms per milliliter, and then the diluted solution was inoculated with a microplanter at the rate of approximately 5 microliters on a MH agar medium containing a test compound. This medium was then incubated at 37° C for 18 hours. The minimum inhibitory concentration (MIC) is determined as a minimum concentration in which no test microorganism could grow. It is noted here that the test organisms used were all standard strains.

Results:

The results are shown in Table 1 below.

Crystalline compound (20) described in Example 11 below was used as the crystalline compound, and amorphous compound (14) obtained in Example 5 below, and cefazolin (CEZ), a cephalosporin compound, and imipenem, a carbapenem compound, which are widely used clinically were used as control compounds.

**Table 1**

MINIMUM INHIBITORY CONCENTRATIONS (MIC)

| Test Organisms | MIC (μg/ml) | | | |
|---|---|---|---|---|
| | Test Compounds | | | |
| | CEZ | Imipenem | Amorphous Compd. (14) | Crystalline Compd. (20) |
| Staphylococcus aureus FDA 209P JC-1 | 0.2 | 0.025 | 0.05 | 0.025 |
| Staphylococcus aureus Terajima | 0.05 | <0.006 | 0.013 | <0.006 |
| Staphylococcus aureus MS352 | 0.1 | 0.013 | 0.05 | 0.025 |
| Streptococcus pyogenes Cook | 0.1 | <0.006 | 0.025 | <0.006 |
| Micrococcus luteus ATCC9341 | 0.39 | 0.025 | 0.1 | - |
| Basillus subtilis ATCC6633 | 0.1 | 0.025 | 0.1 | 0.1 |
| Escherichia coli NIHJ JC-2 | 0.78 | 0.1 | 0.05 | 0.05 |
| Escherichia coli K12 C600 | 0.78 | 3.13 | 1.56 | 0.05 |
| Enterobacter aerougenes ATCC13048 | >100 | 3.13 | 1.56 | 0.78 |
| Enterobacter cloacae 963 | >100 | 0.2 | 0.1 | 0.05 |
| Klebsiella pneumoniae PCI-602 | 0.78 | 0.39 | 0.39 | 0.05 |
| Salmonella typhimurium IID971 | 0.78 | 0.39 | 0.05 | 0.05 |

- to be continued -

**Table 1 (continued)**

| Test Organisms | MIC (μg/ml) Test Compounds | | | |
|---|---|---|---|---|
| | CEZ | Imipenem | Amorphous Compd. (14) | Crystalline Compd. (20) |
| Salmonella typhi 901 | 0.78 | 0.1 | 0.025 | 0.013 |
| Salmonella paratyphi 1015 | 1.56 | 1.56 | 1.56 | 1.56 |
| Salmonella schottmuelleri 8006 | 0.78 | 0.78 | 0.39 | 0.39 |
| Salmonella enteritidis G14 | 0.78 | 0.78 | 1.56 | 0.78 |
| Serratia marcescens IAM1184 | >100 | 0.39 | 0.2 | 0.78 |
| Morganella morganii IFO3848 | 25 | 0.39 | 0.39 | 0.2 |
| Proteus mirabilis IFO3849 | 6.25 | 6.25 | 6.25 | 3.13 |
| Proteus vulgaris OX-19 | 6.25 | 1.56 | 1.56 | 1.56 |
| Proteus vulgaris HX-19 | 6.25 | 1.56 | 1.56 | 1.56 |
| Providencia rettgeri IFO 3850 | 12.5 | 0.78 | 0.05 | 0.025 |
| Pseudomonas aeruginosa IFO 3445 | >100 | 0.78 | 0.39 | 0.39 |
| Pseudomanas aeruginosa NCTC 10940 | >100 | 0.78 | 0.39 | 0.2 |

The foregoing results clearly demonstrate that the carbapenem compound according to the present invention has superior antibacterial activities.

II. Antibacterial Activities against Clinically Isolated β-Lactamase (Cephalosporinase) Producing Strains

Test Procedures:

The antibacterial activities against clinically isolated β-lactamase producing strains were tested by the agar plate dilution method in accordance with the standard method of The Japanese Chemotherapy Society [Chemotherapy, Vol. 29, 76-79 (1981)]. A solution of a cephalosporinase producing strain stored by Episome Research Institute, prepared by incubating the strain in a sensitivity test broth (STB; product of Nissui K. K.) for 18 hours, was diluted with a fresh STB solution so that the solution contained $10^6$ cells per milliliter. The diluted solution was then inoculated as spots in a microplanter on a sensitivity disk agar-N (SDA; product of Nissui K. K.) containing a test compound. The disk agar was then incubated for 18 to 20 hours. The minimum inhibitory concentration was determined as a minimum concentration in which the test microorganism no longer grew after a 18 to 20-hour incubation.

Results:

Table 2 shows the test results. The test compound used therein was the crystalline compound (20) obtained in Example 11. As control compounds, there were used the amorphous compound (14) obtained in Example 5, ceftazidime (CAZ) as a cephalosporin compound, and imipenem as a carbapenem compound, both being recognized as having remarkably high antibacterial activities against the test strains and being widely employed clinically.

Table 2

| Test Organisms | MIC (μg/ml) Test Compounds | | | |
|---|---|---|---|---|
| | CEZ | Imipenem | Amorphous Compd. (14) | Crystalline Compd. (20) |
| Providencia rettgeri GN 5284 | 0.2 | 0.39 | 0.39 | 0.39 |
| Providencia rettgeri GN 4430 | 0.2 | 0.39 | 0.39 | 0.39 |
| Providencia rettgeri GN 4762 | 0.78 | 0.39 | 0.39 | 0.2 |
| Escherichia coli GN 5482 | 0.78 | 0.1 | 0.05 | 0.05 |
| Escherichia coli No. 1501 | 0.2 | 0.1 | 0.05 | 0.05 |
| Escherichia coli No. 96 | 0.78 | 0.1 | 0.05 | 0.05 |
| Enterobacter cloacae GN 7471 | 3.13 | 0.2 | 0.1 | 0.1 |
| Enterobacter cloacae GN 7467 | 3.13 | 0.78 | 3.13 | 1.56 |
| Enterobacter cloacae GN 5797 | 0.39 | 0.39 | 0.1 | 0.1 |
| Proteus morganii GN 5407 | 0.39 | 3.13 | 6.25 | 1.56 |
| Proteus morganii GN 5307 | 0.2 | 0.78 | 3.13 | 0.78 |
| Proteus morganii GN 5375 | 0.2 | 3.13 | 3.13 | 1.56 |
| Proteus vulgaris GN 76 | 0.1 | 3.13 | 6.25 | 3.13 |

- to be continued -

Table 2 (continued)

| Test Organisms | MIC (µg/ml) Test Compounds | | | |
|---|---|---|---|---|
| | CEZ | Imipenem | Amorphous Compd. (14) | Crystalline Compd. (20) |
| Proteus vulgaris GN 7919 | 3.13 | 0.39 | 6.25 | 0.78 |
| Proteus vulgaris GN 4413 | 0.2 | 6.25 | 6.25 | 3.13 |
| Pseudomonas aeruginosa GN 918 | 6.25 | 0.78 | 0.78 | 1.56 |
| Pseudomonas aeruginosa GN 10362 | 3.13 | 0.78 | 0.78 | 0.78 |
| Pseudomonas aeruginosa GN 10367 | 3.13 | 1.56 | 1.56 | 3.13 |
| Serratia marcescens GN 10857 | 0.78 | 3.13 | 6.25 | 3.13 |
| Serratia marcescens L-65 | 0.2 | 0.39 | 0.78 | 0.39 |
| Serratia marcescens L-82 | 0.39 | 0.2 | 0.2 | 0.2 |
| Citrobactor freundii GN 346 | 25 | 0.39 | 0.78 | 0.78 |
| Citrobactor freundii GN 7391 | >100 | 0.78 | 1.56 | 0.78 |
| Pseudomonas cepacia GN 11164 | 0.78 | 3.13 | 0.39 | 0.39 |
| Klebsiella oxytoca GN 10650 | 0.2 | 0.2 | 0.39 | 0.2 |

The above results show that the crystalline carbapenem compound according to the present invention had the antibacterial activities against P. aeruginosa and P. cepacia belonging to Pseudomonadaceae as high as imipenem and particularly higher than CAZ having anti-Pseudomonas activities.

It has been further found that the crystalline carbapenem compound of the invention had activities against enteric bacteria excluding the genus Proteus as high as imipenem and superior to CAZ.

III. Sensitivity Tests against Clinical Isolates

1. P. aeruginosa resistant strains

(1) Strains of Test Organisms:

Fifty-four strains of P. aeruginosa demonstrating resistance against the following agents in the concentrations indicated in the following parentheses were employed for sensitivity tests against clinical isolates.

| | | |
|---|---|---|
| Ceftazidime (CAZ) | (25 to 100 µg/ml) | 21 strains |
| Cefsulodine (CFS) | (25 to >100 µg/ml) | 23 strains |
| Piperacilin (PIPC) | (25 to >100 µg/ml) | 15 strains |
| Gentamycin (GM) | (25 to >100 µg/ml) | 21 strains |
| Amikacin (AMK) | (25 to >100 µg/ml) | 26 strains |
| Ofloxacin (OFLX) | (25 to >100 µg/ml) | 4 strains |

(2) Test Procedures:

The test procedures were based on the agar plate dilution method in accordance with the standard method of The Japanese Chemotherapy Society. The minimum inhibitory concentration (MIC) was determined in substantially the same manner as the test procedures II above using the 54 strains of P. aeruginosa having an anti-Pseudomonas resistance.

(3) Results:

The crystalline compound (20) obtained in Example 11 was found to demonstrate antibacterial activities to inhibit the growth of approximately 99% of the test microorganisms in a concentration of 3.13 µg/ml and all the test microorganisms in a concentration of 6.25 µg/ml.

The imipenem, on the other hand, was found to inhibit the growth of approximately 98% of the test microorganisms in a concentration of 6.25 µg/ml and all the test microorganism in a concentration of 12.5 µg/ml.

The above results clearly demonstrate that the crystalline compound according to the present invention has superior antibacterial activities to imipenem.

IV. Stability Test against Kidney Dehydropeptidase:

1. Materials:

(1) Swine Kidney Dehydropeptidase-I (DHP-I):

The swine kidney (8 kg) was homogenized and the enzyme protein was allowed to precipitate. After a connective lipid was removed with acetone, the resultant material was made soluble by treatment with butanol and purified by the ammonium sulfate fraction method, thereby producing DHP-I enzyme from a 75% ammonium sulfate fraction.

The DHP-I enzyme was then adjusted to give an enzyme concentration of 25 mg/10 ml (phosphate buffer, pH 7.1), and divided into 1 ml portions. The portions were frozen and stored at -40°C or less until use.

(2) Test Compound:

Crystalline compound (20) obtained in Example 11 and amorphous compound (14) obtained in Example 5 below were used as a test compound.

The test compound was adjusted in situ to give a concentration of 117 µM with a 50 mM sodium phosphate buffer solution (pH = 7.1).

Glycyl dehydrophenylalanine (Gl-dh-Ph) and imipenem were employed as control compounds, and they were adjusted in situ each to give a concentration of 117 μM with the same sodium phosphate buffer solution.

2. Method:

(1) Measurement for Hydrolysis Activity against DHP-I Enzyme Substrate by Late Assay:

To 1.2 ml of 50 mM sodium phosphate buffer solution (substrate) containing 117 μM of each of Gl-dh-Ph and imipenem as the control compounds was added 0.2 ml of the DHP-I enzyme solution (25 mg/10 ml) prepared above in the final substrate concentration of 100 μM. The solution was then incubated at 37°C for 10 minutes. The initial velocity of hydrolysis of the substrate was measured from a decrease in absorbency at a particular λmax of each of the substrates.

A blank test was conducted in substantially the same manner as above by adding 0.2 ml of the sodium phosphate buffer solution (pH 7.1) to 1.2 ml of the above substrate.

(2) Measurement for Stability of Test Compounds against DHP-I by High Performance Liquid Chromatography Method (HPLC):

The test compound according to the present invention and the control compounds were treated in substantially the same manner as (1) above. The incubation, however, was conducted at 37°C for 4.5 hours or for 24 hours. The degree of hydrolysis of the compounds each after the test periods was measured by the HPLC method.

3. Results:

The initial velocity of hydrolysis of each of the substrates against DHP-I by the late assay was found as follows:

Gl-dh-Ph: 17.4 μM/minute
Imipenem: 0.56 μM/minute

Table 3 below shows measurement results on stability of the compound according to the present invention and imipenem against DHP-I.

**Table 3**

**DEGREES OF HYDROLYSIS BY DHP-I**

**(Method: HPLC; Substrate Concentration: 100 μM; Unit: μM)**

| | | Test Compounds | |
|---|---|---|---|
| Incubation Conditions | Imipenem | Amorphous Compound (14) | Crystalline Compound (20) |
| 37°C, 4.5 hours | 77.6 | 0.0 | 0.0 |
| 37°C, 24 hours | * | 1.9 | 1.5 |

* After 24 hours at 37°C, it has been found that most or all imipenem has been decomposed and nothing remained was detected.

The stability test results against DHP-I clearly show that the carbapenem compound according to the present invention was several tens of times as stable as imipenem.

V. Toxicity:

Toxicological studies were carried out using a group of 10 male mice of CrjCD(SD) strain weighing from 20 to 23 grams. A solution containing the crystalline carbapenem compound (20) of the present invention obtained by Example 11 was administered intravenously to the mice and subjected to observations for one week.

The results have revealed that the group of mice to which the crystalline carbapenem compound (20) of the present invention had been administered in the amount of 500 mg/kg were alive without any abnormal observations.

As described above, the crystalline carbapenem compound according to the present invention demonstrates a wider scope of antibacterial spectra than do conventional cephalosporin compounds, and remarkable antibacterial activities comparable to imipenem as well as an overwhelmingly higher resistance against DHP than imipenem. The crystalline carbapenem compound according to the present invention further possesses antibacterial activities against clinically isolated strains and present favorable effects on infection preventive tests on mice against various organisms.

Therefore, the crystalline carbapenem compound of formula (I-1) according to the present invention permits a single administration without combination with any other compounds and without a risk of any side effect that might be caused in their combined use with a DHP inhibitor, unlike imipenem that was led for the first time to a practically useful antibacterial agent in combination with cilastatin acting as a DHP inhibitor. The carbapenem compound is accordingly extremely useful as antibacterial agent for therapy and prevention of infectious diseases from various pathogenic organisms.

The crystalline carbapenem compound of formula (I-1) according to the present invention may be administered as an antibacterial agent to the human being and other mammalian animals in the form of a pharmaceutically acceptable composition containing an antibacterially effective amount thereof. The administration dose may vary in a wide range with ages, patients, weights and conditions of patients, forms or routes of administration or physicians' diagnoses and may be orally, parenterally or topically administered, to adult patients usually in a standard daily dose range from approximately 200 to approximately 3,000 mg once or in several installments per day.

The pharmaceutically acceptable composition of the crystalline carbapenem compound of formula (I-1) according to the present invention may contain an inorganic or organic, solid or liquid carrier or diluent, which is conventionally used for preparations of medicines, particularly antibiotic preparations, such as an excipient, e.g., starch, lactose, white sugar, crystalline cellulose or calcium hydrogen phosphate; a binder, e.g., acacia, hydroxypropyl cellulose, alginic acid, gelatin or polyvinyl pyrrolidone; a lubricant, e.g., stearic acid, magnesium stearate, calcium stearate, talc or hydrogenated vegetable oil; a disintegrator, e.g., modified starch, calcium carboxymethyl cellulose or low substituted hydroxypropyl cellulose; or a dissolution aid, e.g., a non-ionic surface active agent or an anionic surface active agent, and may be prepared into forms suitable for oral, parenteral or topical administration. The formulations for oral administration may include solid preparations such as tablets, coatings, capsules, troches, powders, fine powders, granules or dry syrups or liquid preparations such as syrups; the formulations for parenteral administration may include, for example, injectable solutions, drip-feed solutions or depositories; and the formulations for topical administration may include, for example, ointments, tinctures, creams or gels. These formulations may be formed by procedures known per se to those skilled in the art in the field of pharmaceutical formulations.

The crystalline carbapenem compound of formula (I-1) according to the present invention is suitably administered in the form of parenteral formulations, particularly in the form of injectable solutions.

The production of the crystalline carbapenem compound of the formula (I-1) according to the present invention will be described more in detail by way of working examples.

In the following description, the following symbols are used to have the particular meanings.

| | |
|---|---|
| ph | : phenyl group |
| PNB | : p-nitrobenzyl group |
| PNZ | : p-nitrobenzyloxycarbonyl group |
| ╁Si | : tertiary-butyldimethylsilyl group |
| Ac | : acetyl group |
| Et | : ethyl group |

Example 1: Production of 1,2,3-Thiadiazol-4-ylmethanol [Compound (1)]

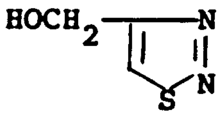

(1)

(a) Ethyl pyruvate (5 g) was dissolved in 5 ml of ethanol, and a solution of 4.5 g of carboethoxyhydrazine in 12 ml of ethanol was gradually added dropwise to the solution of ethyl pyruvate in ethanol. After the mixture was stirred for 15 minutes at room temperature, the solvent was removed from it under reduced pressure to give 8.6 g (98.6%) of ethyl α-N-carbethoxyhydrazonopropionate.

(b) The compound (8.6 g) obtained in the step (a) above was dissolved in 22 ml of thionyl chloride and the solution was refluxed at 70 ° C for 3 hours.

After the thionyl chloride was removed under reduced pressure, the residue was dissolved in 150 ml of benzene and washed with a 5% sodium hydrogen carbonate aqueous solution and then with sodium chloride solution until the solution became neutral in pH. The resulting solution was dried over magnesium sulfate and the solvent was removed under reduced pressure, leaving 6.1 g (91.1%) of ethyl 1,2,3-thiadiazol-4-ylcarboxylate as pale yellow crystals.

NMR ($CDCl_3$)δ: 1.52 (3H, t, J = 7.6Hz), 4.54 (2H, q, J = 7.6Hz), 9.25 (1H, s).

(c) After 1.6 g of lithium aluminum hydride was gradually added to 100 ml of anhydrous ether, a solution of 6.1 g of ethyl 1,2,3-thiadiazol-4-ylcarboxylate obtained in the step (b) above in 70 ml of ether was gradually added dropwise to the resulting solution. The mixture was stirred at room temperature for 18 hours and 8.0 ml of ice water was added to the reaction mixture, followed by further addition of 9.0 ml of a 20% sulfuric acid. After the organic layer was separated, the aqueous layer was extracted with 200 ml of an ethyl acetate/tetrahydrofuran (1/1) mixture. This aqueous layer was combined with the organic layer previously separated. After the combined layer was dried over magnesium sulfate, the solvent was removed under reduced pressure and the resulting residue was purified by silica gel column chromatography (chloroform:ethyl acetate = 1:1) to give 1.2 g (27.3%) of Compound 1 as a yellow oily material.

NMR ($CDCl_3$) δ : 2.48 (1H, bs), 5.22 (2H, s), 8.51 (1H, s).

Example 2: Production of 4-Mercaptomethyl-1,2,3-thiadiazole [Compound (2)]

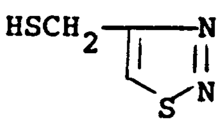

(2)

(a) 1,2,3-Thiadiazol-4-ylmethanol (1.2 g) obtained in Example 1 was dissolved in 70 ml of dichloromethane, and 2.9 ml of triethylamine was added dropwise to the resulting solution. After the solution was cooled to 0 ° C, 1.6 ml of methanesulfonyl chloride was gradually added dropwise. The reaction mixture was stirred at room temperature for 1 hour and 70 ml of dichloromethane was added thereto. After washed twice with a 35 ml portion of water and once with 35 ml of an aqueous sodium chloride solution, the resulting solution was dried over magnesium sulfate and the solvent was removed under reduced pressure. The residue was then dissolved in 160 ml of acetone and 2.4 g (21.1 mmol) of potassium thioacetate was added thereto. After the solution was stirred at room temperature for 18 hours and the acetone was removed under reduced pressure, the residue was then dissolved in 150 ml of dichloromethane and washed twice with a 40 ml portion of water and with 40 ml of an aqueous sodium chloride solution. The solution was then dried over magnesium sulfate and the solvent was removed under reduced pressure, leaving the residue that was in turn purified by silica gel column chromatography (benzene:ethyl acetate = 50:1), yielding 1.2 g (73.1%) of 4-acetylthiomethyl-1,2,3-thiadiazole as a yellow oily material.

NMR ($CDCl_3$) δ : 2.37 (3H, s), 4.58 (2H, s), 8.43 (1H, s).

(b) The compound (1.2 g) obtained in the step (a) above was dissolved in 55 ml of methanol under a nitrogen stream and the solution was cooled to 0 ° C. To the cooled solution was dropwise added 13.3 ml

of a methanol solution of sodium methoxide (28 mg/ml), and the mixture was stirred at 0 ° C for 15 minutes. After addition of 250 ml of dichloromethane, the reaction mixture was washed with 90 ml of 10% hydrochloric acid and then with 50 ml of an aqueous sodium chloride solution. The solution was dried over magnesium sulfate and the solvent was removed under reduced pressure, leaving the residue that was in turn purified by silica gel column chromatography with a chloroform:ethyl acetate (50:1) mixture to give 666 mg (73.1%) of Compound (2) as a yellow oily material.

NMR ($CDCl_3$) $\delta$ : 2.13 (1H, t, J = 8.9Hz), 4.24 (2H, d, J = 8.9Hz), 8.40 (1H, s).

Example 3:

(A)

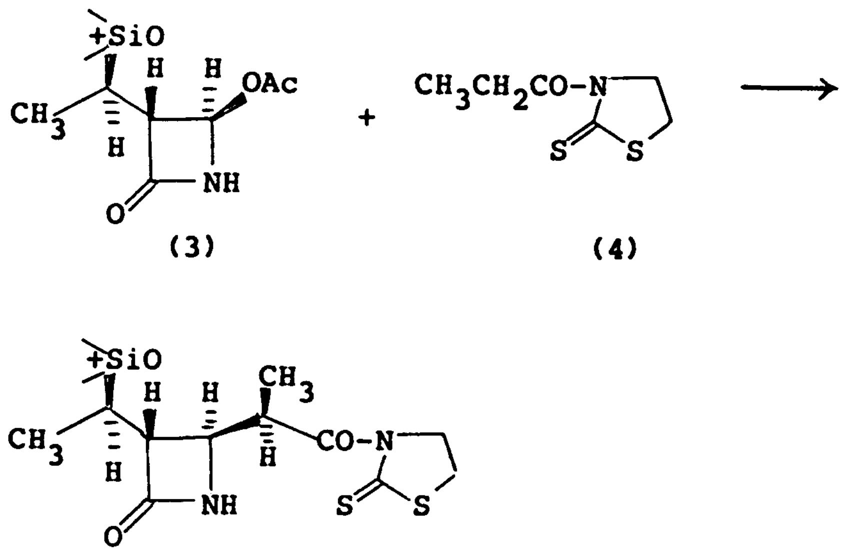

(3) (4)

(5)

Tin triflate (3.712 g) was dissolved in 10 ml of anhydrous tetrahydrofuran under a nitrogen gas stream, and the resulting solution was cooled to 0 ° C. To this solution were added 1.3 ml of N-ethylpiperidine and a solution of 1.2 g of Compound (4) above in 7 ml of anhydrous tetrahydrofuran. The mixture was stirred for 2 hours at the above temperature. A solution of 1.42 g of Compound (3) in 2 ml of anhydrous tetrahydrofuran was added, and the resultant mixture was stirred for 1 hour. After the completion of the reaction, 100 ml of chloroform was added and the mixture was washed with a 10% cirtic acid aqueous solution. The organic layer separated was then dried over $MgSO_4$ and the solvent was removed. The residue was purified by silica gel column chromatography with a n-hexane:ethyl acetate (2-1:1) mixture to give 1.93 g (97%) of Compound (5) as a yellow solid.

NMR ($CDCl_3$) $\delta$ : 0.07 (6H, s), 0.88 (9H, s), 1.21 (3H, d), 1.26 (3H, d), 3.30 (1H, dd), 3.38 (2H, t), 3.94 (1H, dd), 4.55 (2H, t), 6.24 (1H, bs).

(B)

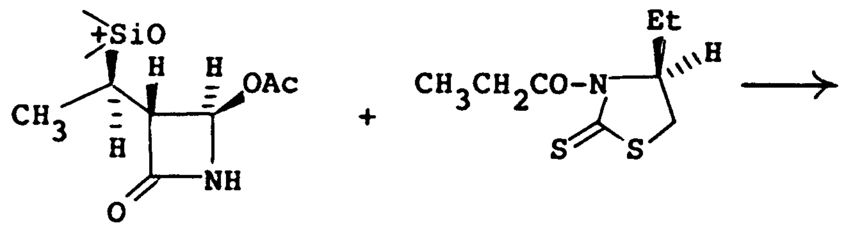

(3) (6)

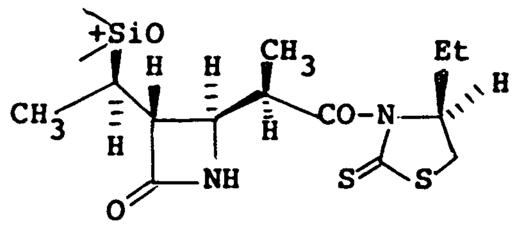

(7)

Tin triflate (57.0 g) was dissolved in 164 ml of anhydrous tetrahydrofuran under a nitrogen gas stream, and the resulting solution was cooled to 0 ° C. To this solution were added 19.9 ml of N-ethylpiperidine and a solution of 21.71 g of Compound (6) above in 123 ml of anhydrous tetrahydrofuran. The mixture was stirred for 1.5 hours at the above temperature. A solution of 1.42 g of Compound (3) in 123 ml of anhydrous tetrahydrofuran was added, and the resultant mixture was stirred for 1 hour. After the completion of the reaction, chloroform was added and the mixture was washed with a 10% citric acid aqueous solution and a sodium chloride aqueous solution. The organic solution separated was then dried over $MgSO_4$ and the solvent was removed. The residue was purified by silica gel column chromatography with n-hexane:ethyl acetate (2:1) to give 33.57 g (98%) of Compound (7) as a yellow solid, m.p. 85.5-86.5 ° C.

NMR ($CDCl_3$) $\delta$: 0.07 (6H, s), 0.90 (9H, s), 1.00 (3H, t), 1.23 (3H, d), 1.26 (3H, d), 2.90 (1H, dd), 3.50 (1H, dd), 6.10 (1H, bs).

$[\alpha]_D^{25}$ = +233.9 ° (c = 0.77, $CHCl_3$).

(C)

Compound (7) 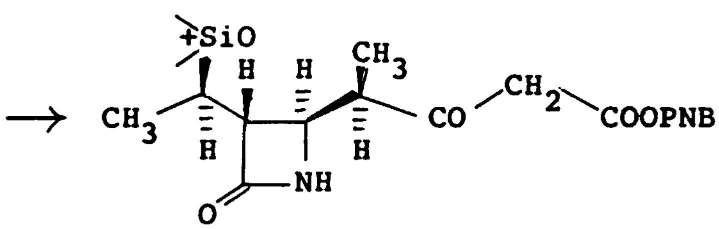

(8)

To a solution of 30.66 g of Compound (7) obtained in the step (B) above in 740 ml of anhydrous acetonitrile was added 12.13 g of imidazole, and the mixture was stirred under a nitrogen gas stream at room temperature for 5.5 hours. Then, 53.39 g of $Mg(O_2CCH_2CO_2PNB)_2$ was added, and the mixture was stirred overnight at 60 ° C. The resultant reaction mixture was concentrated under reduced pressure to 200 ml and 1 liter of ethyl acetate was added thereto. The organic layer separated was washed with a 1N-HCl aqueous solution, a 5% aqueous $NaHCO_3$ solution and an aqueous sodium chloride solution in this order. After drying over $MgSO_4$, the solvent was removed and the residue was purified by column chromatography with 800 g of silica gel to yield 34.47 g of Compound (8) as a colorless oil.

NMR ($CDCl_3$) $\delta$: 0.06 (6H, s), 0.87 (9H, s), 1.16 (3H, d), 1.20 (3H, d), 3.63 (2H, s), 5.27 (2H, s), 5.92 (1H, bs), 7.56, 8.24 (4H aromatic ring proton)

Compound (8) obtained above was used in the following step (D) without further purification.

(D)

Compound (8)

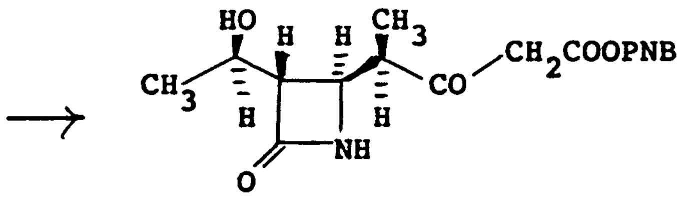

(9)

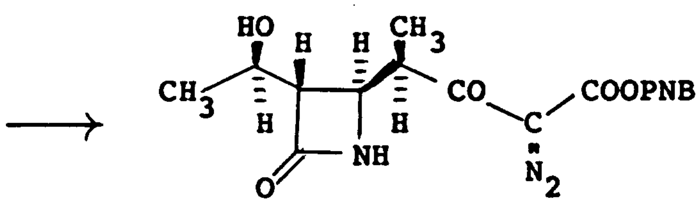

(10)

To a solution of 37.47 g of Compound (8) obtained in the step (C) above in 392 ml of methanol was added 19.6 ml of concentrated HCl, and the mixture was stirred at room temperature for 1.5 hours. The reaction mixture was concentrated to approximately 100 ml, and 800 ml of ethyl acetate was added. After the mixture was washed with water and then with an aqueous sodium chloride solution, it was then dried over $MgSO_4$. The solvent was removed under reduced pressure to yield Compound (9) as a colorless oil.

NMR ($CDCl_3$)$\delta$ : 1.25 (3H, d), 1.30 (3H, d), 2.90 (2H, m), 3.65 (2H, s), 3.83 (1H, m), 4.15 (1H, m), 5.27 (2H, s), 6.03 (1H, bs), 7.55, 8.27 (4H, aromatic ring proton)

Compound (9) was then dissolved in 408 ml of anhydrous acetonitrile, and 36.31 g of dodecylbenzenesulfonyl azide and 13.81 ml of triethylamine were added. After the mixture was stirred at room temperature for 20 minutes, the solvent was removed. The residue was purified by column chromatography with 800 g of silica gel using chloroform:acetone (2:1) to give 21.57 g [69.4% as total yields of Compounds (B), (C) and (D)] of Compound (10) as a colorless oil.

IR ($CHCl_3$) $cm^{-1}$: 2150, 1750, 1720, 1650.

NMR ($CDCl_3$)$\delta$ : 1.23 (3H, d), 1.30 (3H, d), 2.92 (1H, m), 3.50-4.30 (3H, m), 5.38 (2H, s), 6.40 (1H, bs), 7.57, 8.30 (4H, aromatic ring proton)

$[\alpha]_D^{21}$ = -41.6° (c=3.1, $CH_2Cl_2$).

(E)

Compound (10)

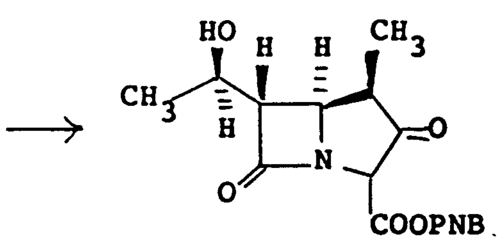

(11)

In 134 ml of ethyl acetate was dissolved 21.57 g of Compound (10) obtained in the step (D) above, and 0.065 g of rhodium octanoate was added. The solution was stirred at 80°C for 0.5 hour and the solvent was removed. The residue was dried to give Compound (11) as a solid.

IR ($CHCl_3$) $cm^{-1}$: 2950, 2925, 1860, 1830.

NMR ($CDCl_3$) δ : 1.22 (3H, d, J=8.0Hz), 1.37 (3H, d, J=6.0Hz), 2.40 (1H, bs), 2.83 (3H, q, J=8.0Hz), 3.28 (1H, d, d), 4.00-4.50 (2H, m), 4.75 (1H, s), 5.28 and 5.39 (2H, ABq, J=12Hz), 7.58, 8.24 (4H, aromatic ring proton)

(F)

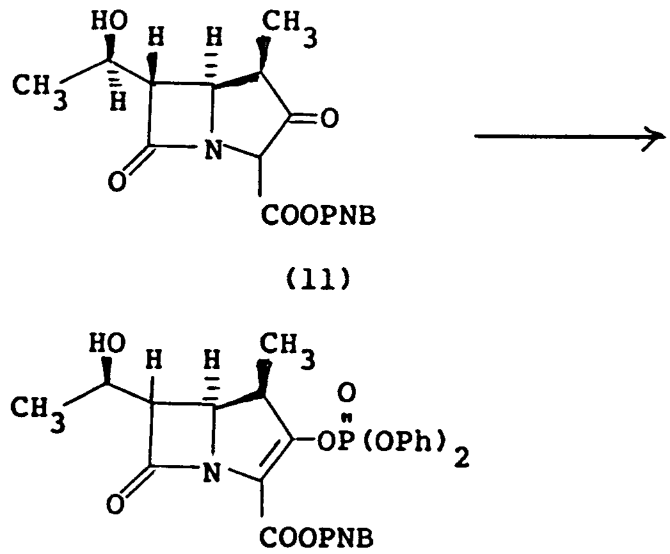

(11)

(12)

To a solution of 186 mg of Compound (11) obtained in the step (E) in 2 ml of anhydrous acetonitrile were added 0.11 ml of diphenylphosphoric chloride and 0.09 ml of diisopropylethyl amine under cooling with ice, and the mixture was stirred for 0.5 hour at the same temperature. After the reaction mixture was concentrated, the residue was purified by silica gel column chromatography to yield 252 mg of Compound (12) as a white solid.

NMR ($CDCl_3$) δ: 1.24 (3H, d), 1.34 (3H, d), 3.30 (1H, q), 3.52 (1H, m), 4.10-4.40 (2H, m), 5.20 and 5.35 (2H, q), 7.29 (1H, m), 7.58 and 8.18 (4H, d).

Example 4:

Compound (12) 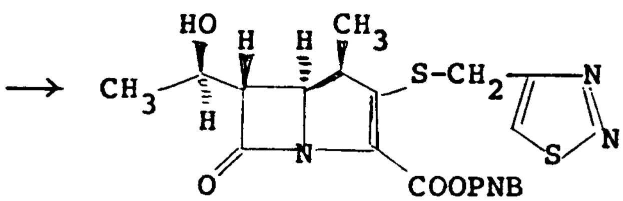 (13)

A solution of 1.7 g of Compound (12) obtained in Example 3 in 15 ml of anhydrous acetonitrile was cooled to -30°C and a solution of 364 mg of Compound (2) obtained in Example 2 in 7 ml of anhydrous acetonitrile was added thereto. To this solution was then added 0.5 ml of diisopropylethyl amine, and the mixture was stirred at 0°C for 1 hour. Removal of the solvent left the residue that was in turn purified by silica gel column chromatography (chloroform:ethyl acetone = 1:1), yielding 1.1 g (80.7%) of Compound (13).

NMR ($CDCl_3$) δ : 1.27 (3H, d, J=7.0Hz), 1.35 (3H, d, J=6.0Hz), 3.25 (1H, dd, J=3.0, 6.0Hz), 3.58-4.65 (5H, m), 5.12 and 5.48 (2H, ABq, J=14.0, 27.0Hz), 7.56-8.25 (8H, m), 8.43 (1H, s).

Example 5:

Compound (13) → 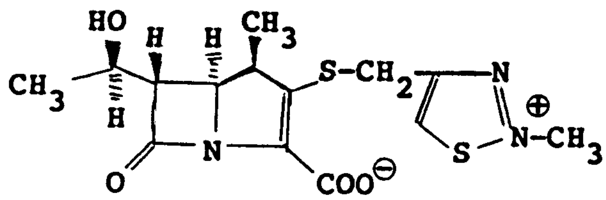 (14)

Compound (13) (257 mg) obtained in Example 4 was dissolved in 4.0 ml of dichloromethane, and the solution was cooled with ice to 0 ° C. After dropwise addition of 0.073 ml of methyl trifluoromethanesulfonate, the mixture was stirred at 0 ° C for 18 hours. To this solution were added 5.0 ml of a 0.5M N-methylmorpholine-HCl buffer solution (pH 6.8), 4.4 ml of n-butanol and 4.0 ml of ethyl acetate in this order. After further addition of 260 mg of a 20% palladium hydroxide-carbon, the catalytic hydrogenation was carried out at room temperature for 2 hours under 3.0 atmospheres, and the reaction mixture was filtered using Celite. After the Celite layer was then washed with small amount of methanol and water, the filtrates were collected and washed with ether. The aqueous layer was then concentrated under reduced pressure and purified on a Dowex 50W-X4($Na^+$) column. The product was then frozen to give 22.6 mg (11.8%) of Compound (14).

IR (KBr) $cm^{-1}$: 1750, 1590, 1380.

NMR ($CD_3OD$) δ: 1.16 (3H, d, J = 7.0Hz), 1.24 (3H, d, J = 6.0Hz), 3.12-3.45 (3H, m), 3.98-4.52 (3H, m), 4.63 (3H, s).

Example 6:

Compound (13) → Compound (14)

In 4 ml of dichloromethane was dissolved 291 mg of Compound (13) obtained in Example 4, and the mixture was cooled to 0 ° C. After dropwise addition of 0.083 ml of methyl trifluoromethanesulfonate, the mixture was stirred at the same temperature for 18 hours. To this mixture were added 20 ml of tetrahydrofuran, 20 ml of ether and 25 ml of a 0.01M phosphate buffer solution (pH 7.0) in this order. After further addition thereto of 330 mg of a 10% palladium-carbon, the mixture were subjected to hydrogenation at room temperature under 3 atmospheres for 1 hour. The reaction mixture was treated in the same manner as in Example 5 to give 66 mg (30.5%) Compound (14).

IR and NMR spectra of this compound correspond fully to those of the compound obtained in Example 5.

Example 7: Production of Compound (14)

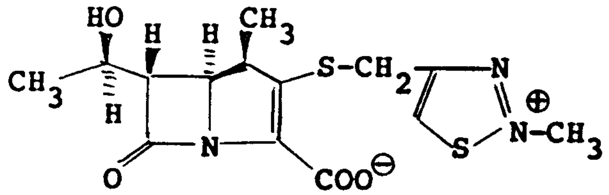

(14)

(a) 4-Tert.-butyldiphenylsilylthiomethyl-1,2,3-thiadiazole [Compound (15)]

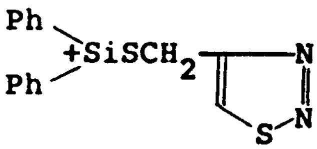

(15)

To a solution of 2.64 g of 4-mercaptomethyl-1,2,3-thiadiazol-4 [Compound (2)] obtained in Example 2 in 40 ml of dichloromethane was added a mixture of 6.5 ml of tert.-butyldiphenylchlorosilane and 3.5 ml of triethylamine at 0 ° C under a nitrogen atmosphere. After the mixture was stirred for 30 minutes at the same temperature, the solvent was removed and the resulting residue was purified by silica gel column chromatography (hexane: ethyl acetate = 3:1) to give 7.4 g of Compound (15) as a pale yellowish oil.

(b) 2-Methyl-4-tert.-butyldiphenylsilylthiomethyl-1,2,3-thiadiazolium triflate [Compound (16)]

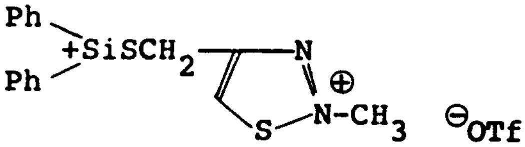

(16)

To a mixture of 7.4 g of Compound (15) obtained in the above step (a) in 15 ml of ether was added 2.8 ml of methyl triflate at 0 ° C under a nitrogen atmosphere and the reaction mixture was stirred for 4 hours. The solvent was then evaporated and the residue was washed with n-hexane to give 9.18 g (86%) of Compound (16) as a pale yellowish oil.

(c) Compound (12) + Compound (16) →

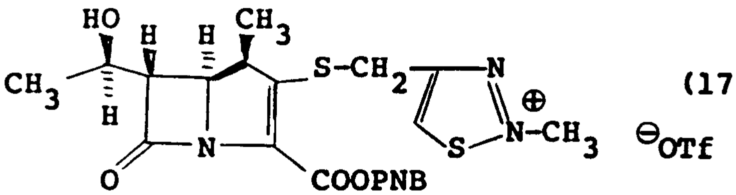

(17)

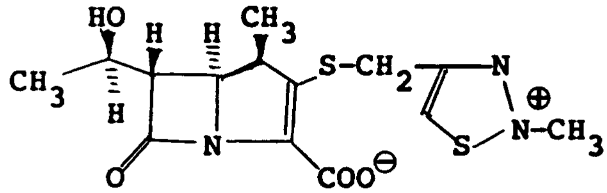

(14)

To a solution of 8.6 g of Compound (12) obtained in Example 3 and 9.18 g of Compound (16) obtained in the above step (b) in a solvent mixture of 26 ml of dimethylacetoamide and 90 ml of acetonitrile was dropwise added 15.6 ml of tetrabutylammonium fluoride (1.0M solution in tetrahydrofuran) at 40 °C under a nitrogen atmosphere and the reaction mixture was stirred for 2 hours at the same temperature. At this stage, Compound (17) was produced. After completion of the reaction, a solvent mixture of 220 ml of 0.35M acetone buffer (pH 5.5), 70 ml of tetrahydrofuran and 150 ml of ether was added. To this was further added 8.5 g of a 10% palladium-carbon, and the mixture was subjected to hydrogenation at room temperature under 4 atmospheres for 40 minutes. After the reaction mixture was filtered using Celite, the water layer was then adjusted to pH 6.8 and concentrated to a smaller volume. The resulting residue was purified using a Dowex 50W-X4($Na^+$) column with water as an eluting solvent, thus yielding 2.16 g (43%) of Compound (14) after lyophilization.

IR and NMR spectra of this compound corresponded fully to those of the compound obtained in Example 5.

Example 8: Production of Compound (14)

Compound (12) + 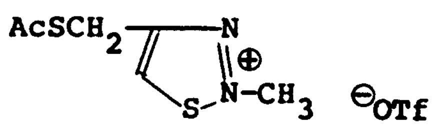 (18)

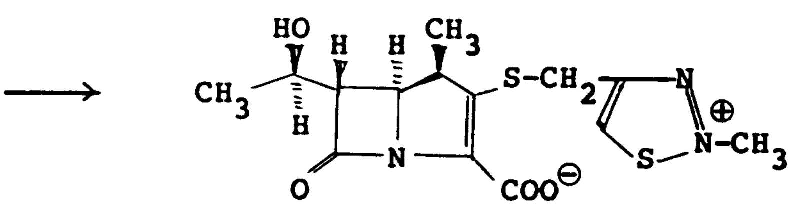

(14)

(a) Production of Compound (18)

To a solution of 4.6 g of 4-acetylthiomethyl-1,2,3-thiadiazole by the above step (a) of Example 2 in a solvent mixture of 26 ml of ether and 2.6 ml of dichloromethane was added 3.3 ml of methyl triflate at 0 °C under a nitrogen atmosphere. After the mixture was stirred for 18 hours at 5 °C, the resulting precipitates were collected and washed with ether to give 8.3 g of Compound (18).

(b) A mixture of 338 mg of Compound (18) obtained in the above step (a), 4 ml of ethanol and 1 ml of water was cooled to -20 ° C. To this mixture was added 1 ml of a 1N sodium hydroxide solution and the mixture was stirred for a few minutes. To the resulting reaction mixture was added a mixture of 298 mg of Compound (12) obtained in Example 4, 10 ml of tetrahydrofuran and 8 ml of 0.18M phosphate buffer, and the mixture was stirred for 1 hour at the same temperature. Then a solution mixture of 20 ml of ether and 10 ml of water was added, and the resulting mixture was subjected to hydrogenation using 2.5 g of a 2% palladium-alumina for 1 hour under 1.5 atmospheres. After completion of the reaction, the reaction mixture was treated in the same manner as have been described in Example 7, thus yielding 59.6 mg (33.2%) of Compound (14).

This compound was identified as the compound obtained in Example 5 by IR and NMR spectra.

Example 9: Production of Compound (14)

(a)

Compound (12) + 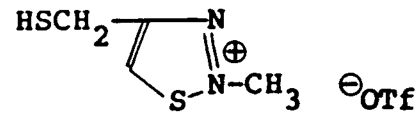 (19)

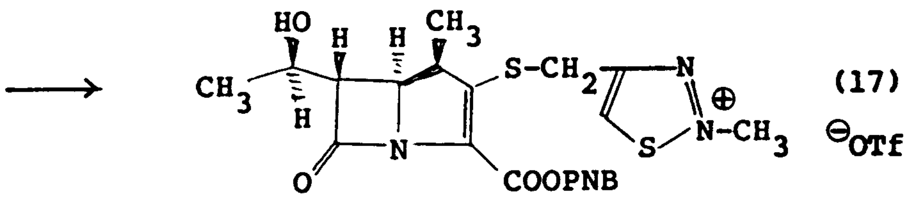 (17)

A mixture of 676 mg of 4-mercaptomethyl-2-methyl-1,2,3-thiadiazolium trifluoromethanesulfonate [Compound (19)], 4 ml of methanol and 1 ml of water was cooled to -20 ° C. To this mixture was added 2 ml of a 1N sodium hydroxide solution and the mixture was stirred for a few minutes. To this mixture was added a solution of 594 mg of Compound (12) obtained in Example 3 in 10 ml of tetrahydrofuran and 8 ml of 0.35M phosphate buffer (pH 7.0) under ice-cooling, and the reaction mixture was stirred for 1 hour at the same temperature to give the Compound (17) in the reaction mixture.

This compound was used for the next step without isolation from the reaction mixture.

(b)

Compound (17) 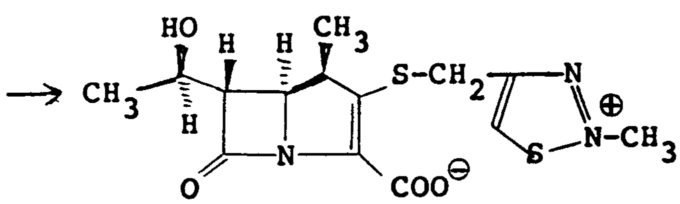 (20)

To the above reaction mixture was added 20 ml of 0.35M phosphate buffer, and a pH of this mixture was adjusted to 6.1 by adding few drops of phosphoric acid. After further addition thereto of 1.2 g of zinc powder, the reaction mixture was stirred for 30 minutes at 18-20 ° C, then the reaction mixture was filtered using Celite. The organic solvent in filtrate was removed off under reduced pressure and the resulting residue was washed with 100 ml of ethyl acetate. The aqueous layer was condensed under reduced pressure and the residue was then adjusted to pH 6.3-65. The resulting residue was purified using Dowex 50-WX4 column with water at eluent to give 195.4 mg (54.4%) of Compound (14) after lyophilization.

This compound was identified as the compound obtained in Example 5 by IR and NMR spectra.

Example 10: Production of Compound (14)

(a) Compound (12) + Compound (16) →

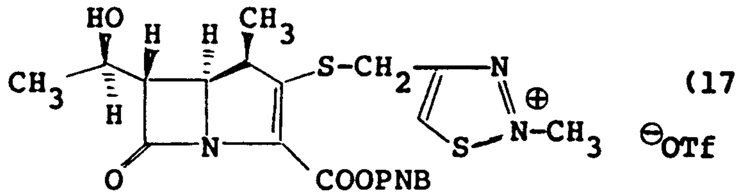

(17)

To a solution of 804 mg of Compound (12) obtained in Example 3 and 940 mg of Compound (16) obtained in the step (b) of Example 7 in 21 ml of acetonitrile was added dropwise a mixture solution of 1.62 ml of tetrabutylammonium fluoride (1.0M solution in tetrahydrofuran) and 2 ml of tetrahydrofuran at -40 °C under a nitrogen atmosphere. After dropwise, the reaction mixture was stirred for 20 minutes at the same temperature to give the Compound (17). This compound was used in the next step without isolation.

(b)

Compound (17) →

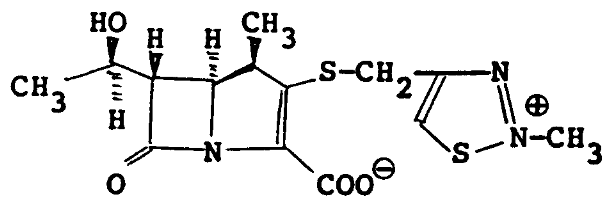

(14)

To the above reaction mixture was added 12 ml of 0.5M phosphate buffer (pH 7.0), 21 ml of water and 1.6 g of zinc powder. Then a pH of this mixture was adjusted to 6-7 by adding a saturated potassium phosphate solution. After stirring was continued for 20 minutes, the reaction mixture was filtered using Celite and the filtrate was washed with 50 ml of ether. The ether layer was extracted 50 ml of water and the combined water layer was adjusted to pH 6.8 and concentrated to a smaller volume. The resulting residue was purified using a Dowex 50W-X4 (Na+) column with water as an eluent to give 251 mg (52%) of the Compound (14) after lyophilization.

This compound was identified as the compound obtained in Example 5 by IR and NMR spectra.

Example 11: Crystalline (1R,5S,6S)-2-[(2-methyl-1,2,3-thiadiazolium-4-yl)methyl]thio-6-[(R)-1-hydroxyethyl]-1-methyl-carbapenem-3-carboxylate [Crystalline compound (20)]

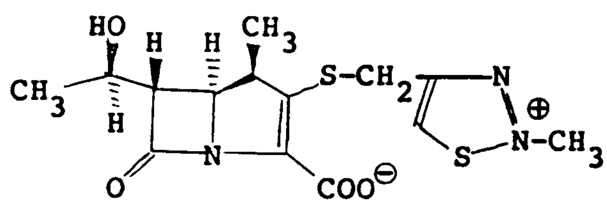

(20)

One gram of amorphous compound (14) obtained in Example 10 (lyophilized product found to be amorphous by observation under a polarizing microscope) was dissolved in 0.5 ml of water, and the solution was stirred at room temperature for 2 hours. With stirring, the solution became homogeneous, and then crystals precipitated. The crystals were collected by filtration, washed with a small amount of ethanol, and dried in vacuum at room temperature for 20 hours to give 251 mg (25.1%) of a crystalline compound (20)

as pale yellowish white crystals.

The crystalline compound (20) was found to be crystalline by observation under a polarizing microscope, and showed characteristic peaks shown in Table 4 in powder X-ray diffractometry.

Table 4

| 2θ | d |
|---|---|
| 12.8 | 6.9 |
| 16.8 | 5.3 |
| 19.2 | 4.6 |
| 21.1 | 4.2 |
| 23.0 | 3.9 |
| 27.2 | 3.3 |
| 30.2 | 3.0 |
| 35.5 | 2.5 |
| 37.6 | 2.4 |

Cu (λ = 1.5418) was used as an X-ray source, and the spacing d (unit Å) was calculated from the following equation.

$$d = \frac{\lambda}{2\sin\theta} = \frac{1.5418}{2\sin\theta}$$

Example 12: Crystalline (1R, 5S, 6S)-2-[(2-methyl-1,2,3-thiadiazolium-4-yl)methyl]thio-6-[(R)-1-hydroxyethyl]-1-methyl-carbapenem-3-carboxylate [Crystalline compound (20)]:

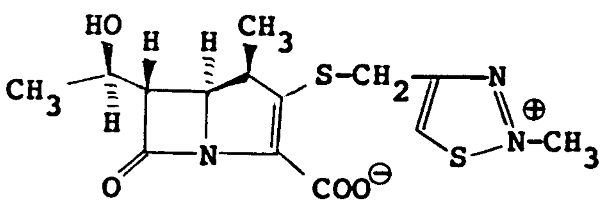

(20)

The crystalline compound (20) obtained in Example 11 was used as seed crystals for crystallization. Specifically, 250 mg of the amorphous compound (14) obtained in Example 10 was dissolved in 0.5 ml of water, and 2.5 ml of ethanol was further added to the solution. A small amount of the crystalline compound (20) obtained in Example 11 was added as seed crystals. When the solution was stirred at room temperature for 2 hours, crystals precipitated. The resulting crystals were collected by filtration, washed with a small amount of ethanol, and dried in vacuum at room temperature for 20 hours to give 142 mg (56.8%) of crystalline compound (20) as pale yellowish crystals.

The crystalline compound (20) was determined to be crystalline by observation under a polarizing microscope.

The crystals obtained in Examples 11 and 12 show the same physical analysis data. The results are summarized below.

## Table 5

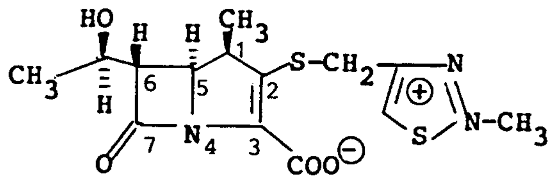

Molecular weight: 355.44
Molecular formula: $C_{14}H_{17}N_3O_4S_2$

| | |
|---|---|
| Elemental analysis | for $C_{14}H_{17}N_3O_4S_2 \cdot 2H_2O$<br>C H N<br>Calculated: 45.03 5.13 11.15<br>Found: 45.03 5.15 11.38 |
| Mass analysis | M/Z: 356 $(M+H)^+$<br><FAB-MS> |
| UV | λmax.: 293 nm ($H_2O$) |
| IR | νmax.:(KBr)$cm^{-1}$: 3320 (br), 1752, 1636, 1598 |
| $^1$H-NMR<br><br>NMR DATA | 400 MHz ($D_2O$)<br>δ:1.25 ($C_1$-$CH_3$), 1.32 ( 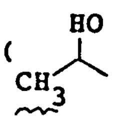 ), 3.41 ($C_1$-H),<br>3.53 ($C_6$-H), 4.24 ($C_5$-H), 4.27 ( 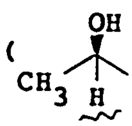 ),<br>4.48, 4.66 ($SCH_2$-)<br>4.71 (N-$CH_3$) |
| $^{13}$C-NMR ($D_2O$) | δ:16.23(1β-C), 20.64 ( 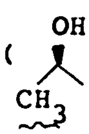 ), 27.10 (-S-$CH_2$-),<br>42.37 ($C_1$), 46.00 ($N^{\oplus}$-C), 56.40 ($C_5$),<br>59.77 ($C_6$), 65.44 ( 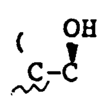 ), 133.60,<br>137.90 ($C_2$, $C_3$), 147.25 ($C_5$'), 155.75 ($C_4$'),<br>167.22 ($C_7$), 177.07 ($COO^{\ominus}$) |

Example 13: Comparison of the stability of Crystalline compound (20) with that of Amorphous compound (14)

Twenty milligrams of each of the crystalline compounds (20) obtained in Examples 11 and 12 and the amorphous compound (14) obtained in Example 10 was put in a glass bottle, and left to stand for 10 days in a constant-temperature chamber at 40° C. The activity of each of the compounds was measured by HPLC after 1, 2, 5 and 10 days from the start of storage. The activity on the first day is taken as 100%, and the decrease of the activity is shown by percent residual activity in Table 6.

Table 6

| PERCENT RESIDUAL ACTIVITY | | | | | |
|---|---|---|---|---|---|
| Compound | Percent residual activity | | | | |
| | first day | after 1 day | after 2 days | after 5 days | after 10 days |
| Crystalline compound (20) of Example 11 | 100 | 100 | 100 | 100 | 100 |
| Crystalline compound (20) of Example 12 | 100 | 100 | 100 | 100 | 100 |
| Amorphous compound (14) of Example 10 | 100 | 90 | 80 | 45 | 35 |

It is clear from the above results that the crystalline compound of this invention has very high stability.

The carbapenem compound according to the present invention may be formulated in various preparation forms.

Formulation Example 1 (Injection):

(1) Injectable suspension:

| | |
|---|---|
| Crystalline compound (20) | 25.0 g |
| Methyl cellulose | 0.5 g |
| Polyvinyl pyrrolidone | 0.05 g |
| Methyl p-oxybenzoate | 0.1 g |
| Polysolvate 80 | 0.1 g |
| Lidocaine hydrochloride | 0.5 g |
| Distilled water | to make 100 ml |

The above components were formulated into 100 ml of an injectable suspension.

(2) Lyophilization:

An appropriate amount of distilled water was added to 20 g of the crystalline compound (20) to make the total volume 100 ml. The above solution (2.5 ml) was filled in vials so as for each vial to contain 500 mg of the crystalline compound (20) and lyophilized. The lyophilized vial was mixed in situ with approximately 3-4 ml of distilled water to make an injectable solution.

(3) Powder:

Crystalline compound (20) was filled in an amount of 250 ml in a vial and mixed in situ with about 3-4 ml of distilled water to make an injectable solution.

Formulation Example 2 (Tablets):

| | |
|---|---|
| Crystalline compound (20) | 250 mg |
| Lactose | 250 mg |
| Hydroxypropyl cellulose | 1 mg |
| Magnesium stearate | 10 mg |
| | 511 mg/tablet |

The above components were mixed with each other and punched into tablets in a conventional manner. Such tablets, as required, may be formulated into sugar coatings or film coatings in a conventional manner.

Formulation Example 3 (Troche):

| | | |
|---|---|---|
| (1) | Crystalline compound (20) | 200 mg |
| | Sugar | 770 mg |
| | Hydroxypropyl cellulose | 5 mg |
| | Magnesium stearate | 20 mg |
| | Flavor | 5 mg |
| | | 1,000 mg/troche |
| (2) | Crystalline compound (20) | 50 mg |
| | Sugar | 240 mg |
| | Hydroxypropyl cellulose | 2 mg |
| | Magnesium stearate | 6 mg |
| | Flavor | 2 mg |
| | | 300 mg/troche |

Each of the components (1) and (2) was mixed with each other and formulated into troches by punching in a conventional manner.

Formulation Example 4 (Capsules):

| | |
|---|---|
| Crystalline compound (20) | 500 mg |
| Magnesium stearate | 10 mg |
| | 510 mg/capsule |

The component was mixed with each other and filled in conventional hard gelatin capsules.

Formulation Example 5 (Dry Syrup):

| | |
|---|---|
| Crystalline compound (20) | 200 mg |
| Hydroxypropyl cellulose | 5 mg |
| Sugar | 793 mg |
| Flavor | 5 mg |
| | 1,000 mg |

The above components were mixed with each other and formulated into dry syrups in a conventional manner.

Formulation Example 6 (Powders):

| | | |
|---|---|---|
| (1) | Crystalline compound (20)<br>Lactose | 200 mg<br>800 mg<br>1,000 mg |
| (2) | Crystalline Compound (20)<br>Lactose | 250 mg<br>750 mg<br>1,000 mg |

Each of the components was mixed with each other and formulated in powders in a conventional manner.

Formulation Example 7 (Suppository):

| | |
|---|---|
| Crystalline compound (20)<br>Witepsol H-12 (Product of Dynamite Noble) | 500 mg<br>700 mg<br>1,200 mg |

The above components were mixed with each other and formulated into suppositories in conventional manner.

## Claims

**Claims for the following Contracting States : AT, BE, CH, DE, FR, GB, IT, LU, NL, SE**

**1.** Crystalline (1R,5S,6S)-2-[(2-methyl-1,2,3-thiadiazolium-4-yl)methyl]thio-6-[(R)-1-hydroxyethyl]-1-methyl-carbapenem-3-carboxylate represented by the following formula:

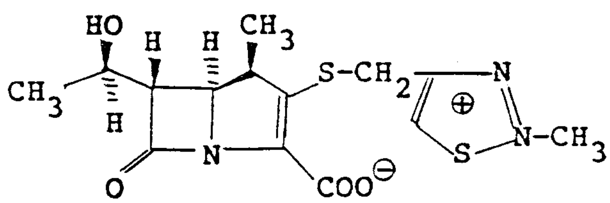

(I-1)

which has characteristic peaks at a spacing (d) of 6.9, 5.3, 4.6, 4.2, 3.9, 3.3, 3.0, 2.5 and 2.4 Å in its powder X-ray diffraction pattern.

**2.** The compound of claim 1 as an antibacterial agent.

**3.** A process for the production of crystalline (1R,5S,6S)-2-[(2-methyl-1,2,3-thiadiazolium-4-yl)methyl]thio-6-[(R)-1-hydroxyethyl]-1-methyl-carbapenem-3-carboxylate represented by the formula:

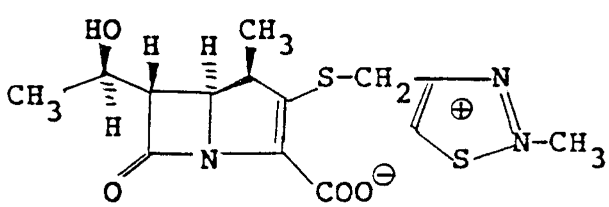

(I-1)

which comprises dissolving amorphous (1R,5S,6S)-2-[(2-methyl-1,2,3-thiadiazolium-4-yl)methyl]thio-6-[-

(R)-1-hydroxyethyl]-1-methyl-carbapenem-3-carboxylate in water in a concentration of at least 65 % and precipitating crystals from the aqueous solution.

**4.** A medicament containing crystalline (1R,5S,6S)-2-[(2-methyl-1,2,3-thiadiazolium-4-yl)methyl]thio-6-[(R)-1-hydroxyethyl]-1-methyl-carbapenem-3-carboxylate of formula (I-1) given in claim 1.

**5.** An antibacterial agent containing crystalline (1R,5S,6S)-2-[(2-methyl-1,2,3-thiadiazolium-4-yl)methyl]thio-6-[(R)-1-hydroxyethyl]-1-methyl-carbapenem-3-carboxylate of formula (I-1) given in claim 1.

**6.** A pharmaceutical composition comprising an antibacterially effective amount of crystalline (1R,5S,6S)-2-[(2-methyl-1,2,3-thiadiazolium-4-yl)methyl]thio-6-[(R)-1-hydroxyethyl]-1-methyl-carbapenem-3-carboxylate of formula (I-1) given in claim 1, and a pharmaceutically acceptable carrier or diluent.

**7.** Crystalline (1R,5S,6S)-2-[(2-methyl-1,2,3-thiadiazolium-4-yl)methyl]thio-6-[(R)-1-hydroxyethyl]-1-methyl-carbapenem-3-carboxylate of formula (I-1) given in claim 1 for the control or prevention of bacterial infections.

**8.** A kit for the provision of a dosage unit of (1R,5S,6S)-2-[(2-methyl-1,2,3-thiadiazolium-4-yl)methyl]thio-6-[(R)-1-hydroxyethyl]-1-methyl-carbapenem-3-carboxylate which comprises a first vial and a second vial, each sealed from contamination by a membrane penetrable by an injection needle, said first vial containing a dosage unit of crystalline (1R,5S,6S)-2-[(2-methyl-1,2,3-thiadiazolium-4-yl)methyl]thio-6-[(R)-1-hydroxyethyl]-1-methyl-carbapenem-3-carboxylate and said second vial containing injectable saline suitable for withdrawal by injection needle and introduction into said first vial to make up a single dosage unit of (1R,5S,6S)-2-[(2-methyl-1,2,3-thiadiazolium-4-yl)methyl]thio-6-[(R)-1-hydroxyethyl]-1-methyl-carbapenem-3-carboxylate.

**9.** A sterile vial sealed from contamination and containing crystalline (1R,5S,6S)-2-[(2-methyl-1,2,3-thiadiazolium-4-yl)methyl]thio-6-[(R)-1-hydroxyethyl]-1-methyl-carbapenem-3-carboxylate, said vial including a membrane which is penetrable by an injection needle.

**Claims for the following Contracting States : ES, GR**

**1.** A process for producing crystalline (1R,5S,6S)-2-[(methyl substituted-1,2,3-thiadiazolium-4-yl)methyl]-thio-6-[(R)-1-hydroxyethyl]-1-methyl-carbapenem-3-carboxylate represented by the following formula:

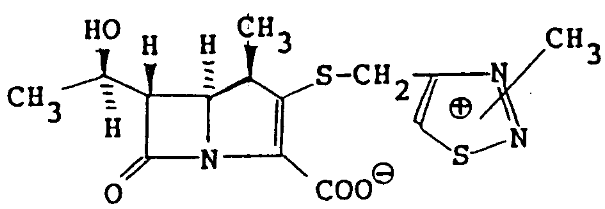

(I)

which comprises dissolving amorphous (1R,5S,6S)-2-[(methyl substituted-1,2,3-thiadiazolium-4-yl)-methyl]thio-6-[(R)-1-hydroxyethyl]-1-methyl-carbapenem-3-carboxylate in water in a concentration of at least 65% and precipitating crystals from the aqueous solution.

**2.** The process of claim 1 for the production of crystalline (1R,5S,6S)-2-[(2-methyl-1,2,3-thiadiazolium-4-yl)methyl]thio-6-[(R)-1-hydroxyethyl]-1-methylcarbapenem-3-carboxylate represented by the formula:

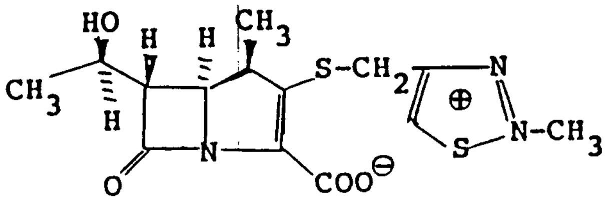

(I-1)

## Patentansprüche

### Patentansprüche für folgende Vertragsstaaten : AT, BE, CH, DE, FR, GB, IT, LI, LU, NL, SE

1. Kristallines (1R,5S,6S)-2-[(2-Methyl-1,2,3-thiadiazolium-4-yl)methyl]thio-6-[(R)-1-hydroxyethyl]-1-methylcarbapenem-3-carboxylat, das durch die folgende Formel:

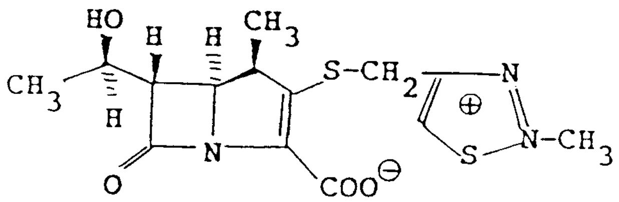

(I-1)

dargestellt wird und das in seinem Pulver-Röntgen-Diffraktionsspektrum charakteristische Peaks bei Abständen (d) von 6,9, 5,3, 4,6, 4,2, 3,9, 3,3, 3,0, 2,5 und 2,4 Å aufweist.

2. Verbindung nach Anspruch 1 als antibakterielles Mittel.

3. Verfahren zur Herstellung von kristallinem (1R,5S,6S)-2-[(2-Methyl-1,2,3-thiadiazolium-4-yl)methyl]thio-6-[(R)-1-hydroxyethyl]-1-methyl-carbapenem-3-carboxylat, dargestellt durch die Formel:

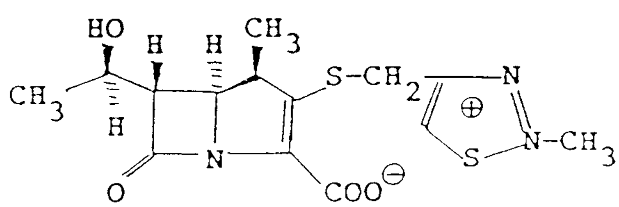

(I-1)

dadurch **gekennzeichnet,** daß amorphes (1R,5S,6S)-2-[(2-Methyl-1,2,3-thiadiazolium-4-yl)methyl]thio-6-[(R)-1-hydroxyethyl]-1-methyl-carbapenem-3-carboxylat in Wasser in einer Konzentration von mindestens 65% aufgelöst wird und aus der wäßrigen Lösung Kristalle ausgefällt werden.

4. Arzneimittel, dadurch **gekennzeichnet,** daß es kristallines (1R,5S,6S)-2-[(2-Methyl-1,2,3-thiadiazolium-4-yl)methyl]thio-6-[(R)-1-hydroxyethyl]-1-methyl-carbapenem-3-carboxylat der in Anspruch 1 angegebenen Formel (I-1) enthält.

5. Antibakterielles Mittel, dadurch **gekennzeichnet,** daß es kristallines (1R,5S,6S)-2-[(2-Methyl-1,2,3-thiadiazolium-4-yl)methyl]thio-6-[(R)-1-hydroxyethyl]-1-methyl-carbapenem-3-carboxylat der in Anspruch 1 angegebenen Formel (I-1) enthält.

6. Pharmazeutisches Präparat, dadurch **gekennzeichnet,** daß es eine antibakteriell wirksame Menge von kristallinem (1R,5S,6S)-2-[(2-Methyl-1,2,3-thiadiazolium-4-yl)methyl]thio-6-[(R)-1-hydroxyethyl]-1-methyl-carbapenem-3-carboxylat der in Anspruch 1 angegebenen Formel (I-1) und einen pharmazeutisch annehmbaren Träger oder ein Verdünnungsmittel enthält.

7. Kristallines (1R,5S,6S)-2-[(2-Methyl-1,2,3-thiadiazolium-4-yl)methyl]thio-6-[(R)-1-hydroxyethyl]-1-methyl-carbapenem-3-carboxylat der in Anspruch 1 angegebenen Formel (I-1) für die Kontrolle oder Prophylaxe von bakteriellen Infektionen.

8. Kit für die Zurverfügungstellung einer Dosiseinheit von (1R,5S,6S)-2-[(2-Methyl-1,2,3-thiadiazolium-4-yl)-methyl]thio-6-[(R)-1-hydroxyethyl]-1-methyl-carbapenem-3-carboxylat, dadurch **gekennzeichnet,** daß es ein erstes Fläschchen und ein zweites Fläschchen umfaßt, wovon jedes von Verunreinigungen mittels einer Membran, durch die eine Injektionsnadel eindringen kann, verschlossen ist, wobei das erste Fläschchen eine Dosiseinheit an kristallinem (1R,5S,6S)-2-[(2-Methyl-1,2,3-thiadiazolium-4-yl)-methyl]thio-6-[(R)-1-hydroxyethyl]-1-methyl-carbapenem-3-carboxylat enthält und das zweite Fläschchen eine injizierbare Salzlösung für die Entnahme mittels einer Injektionsnadel und Einführung in das erste Fläschchen enthält, wodurch eine einzige Dosiseinheit von (1R,5S,6S)-2-[(2-Methyl-1,2,3-thiadiazolium-4-yl)methyl]thio-6-[(R)-1-hydroxyethyl]-1-methyl-carbapenem-3-carboxylat gebildet wird.

9. Steriles Fläschchen, abgesiegelt bzw. abgedichtet von Verunreinigungen, das kristallines (1R,5S,6S)-2-[(2-Methyl-1,2,3-thiadiazolium-4-yl)methyl]thio-6-[(R)-1-hydroxyethyl]-1-methyl-carbapenem-3-carboxylat enthält, wobei das Fläschchen eine Membran umfaßt, durch die eine Injektionsnadel dringen kann.

**Patentansprüche für folgende Vertragsstaaten : ES, GR**

1. Verfahren zur Herstellung von kristallinem (1R,5S,6S)-2-[(Methyl-substituiertem-1,2,3-thiadiazolium-4-yl)-methyl]thio-6-[(R)-1-hydroxyethyl]-1-methyl-carbapenem-3-carboxylat, das durch die folgende Formel:

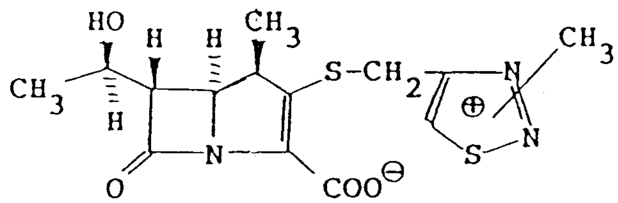

(I)

dargestellt wird, dadurch **gekennzeichnet,** daß amorphes (1R,5S,6S)-2-[(Methyl-substituiertes-1,2,3-thiadiazolium-4-yl)methyl]thio-6-[(R)-1-hydroxyethyl]-1-methyl-carbapenem-3-carboxylat in Wasser in einer Konzentration von mindestens 65% aufgelöst wird und die Kristalle aus der wäßrigen Lösung ausgefällt werden.

2. Verfahren nach Anspruch 1 für die Herstellung von kristallinem (1R,5S,6S)-2-[(2-Methyl-1,2,3-thiadiazolium-4-yl)methyl]thio-6-[(R)-1-hydroxyethyl]-1-methyl-carbapenem-3-carboxylat, dargestellt durch die Formel:

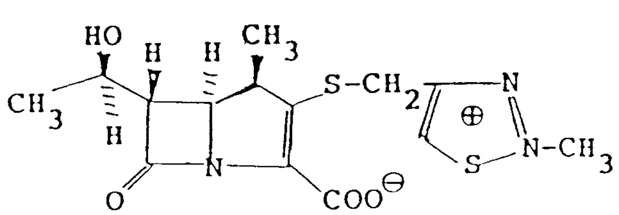

(I-1)

**Revendications**

**Revendications pour les Etats contractants suivants : AT, BE, CH, DE, FR, GB, IT, LU, NL, SE**

1. (1R,5S,6S)-2-[(2-méthyl-1,2,3-thiadiazolium-4-yl)méthyl]thio-6-[(R)-1-hydroxyéthyl]-1-méthylcarbapénème-3-carboxylate cristallin représenté par la formule suivante :

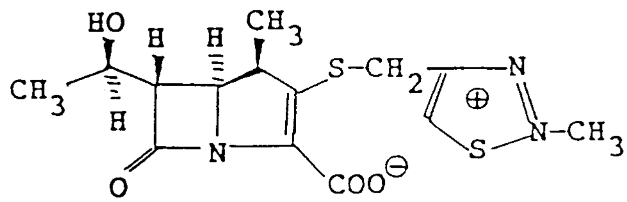

(I-1)

qui présente des pics caractéristiques à des distances (d) de 6,9, 5,3, 4,6, 4,2, 3,9, 3,3, 3,0, 2,5 et 2,4 Å dans son diagramme de diffraction des rayons X par la méthode des poudres.

2. Composé de la revendication 1 comme agent antibactérien.

3. Procédé pour la production de (1R,5S,6S)-2-[(2-méthyl-1,2,3-thiadiazolium-4-yl)méthyl]thio-6-[(R)-1-hydroxyéthyl]-1-méthylcarbapénème-3-carboxylate cristallin représenté par la formule :

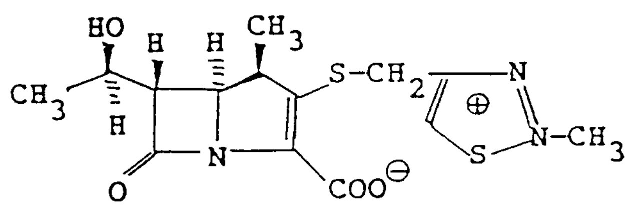

(I-1)

qui consiste à dissoudre le (1R,5S,6S)-2-[(2-méthyl-1,2,3-thiadiazolium-4-yl)méthyl]thio-6-[(R)-1-hydroxyéthyl]-1-méthylcarbapénème-3-carboxylate amorphe dans de l'eau à une concentration d'au moins 65 % et à faire précipiter des cristaux de la solution aqueuse.

4. Médicament contenant du (1R,5S,6S)-2-[(2-méthyl-1,2,3-thiadiazolium-4-yl)méthyl]thio-6-[(R)-1-hydroxyéthyl]-1-méthylcarbapénème-3-carboxylate cristallin de formule (I-1) donnée dans la revendication 1.

5. Agent antibactérien contenant du (1R,5S,6S)-2-[(2-méthyl-1,2,3-thiadiazolium-4-yl)méthyl]thio-6-[(R)-1-hydroxyéthyl]-1-méthylcarbapénème-3-carboxylate cristallin de formule (I-1) donnée dans la revendication 1.

6. Composition pharmaceutique comprenant une quantité antibactérienne efficace de (1R,5S,6S)-2-[(2-méthyl-1,2,3-thiadiazolium-4-yl)méthyl]thio-6-[(R)-1-hydroxyéthyl]-1-méthylcarbapénème-3-carboxylate cristallin de formule (I-1) donnée dans la revendication 1, et un support ou diluant pharmaceutiquement acceptable.

7. (1R,5S,6S)-2-[(2-méthyl-1,2,3-thiadiazolium-4-yl)méthyl]thio-6-[(R)-1-hydroxyéthyl]-1-méthylcarbapénème-3-carboxylate cristallin de formule (I-1) donnée dans la revendication 1 pour combattre ou prévenir des infections bactériennes.

8. Nécessaire pour la fourniture d'une unité posologique de (1R,5S,6S)-2-[(2-méthyl-1,2,3-thiadiazolium-4-yl)méthyl]thio-6-[(R)-1-hydroxyéthyl]-1-méthylcarbapénème-3-carboxylate, qui comprend un premier flacon et un second flacon, chacun étant bouché de façon à empêcher une contamination au moyen d'une membrane dans laquelle peut pénétrer une aiguille d'injection, ledit premier flacon contenant une unité posologique de (1R,5S,6S)-2-[(2-méthyl-1,2,3-thiadiazolium-4-yl)méthyl]thio-6-[(R)-1-hydroxyéthyl]-1-méthylcarbapénème-3-carboxylate cristallin et ledit second flacon contenant une solution saline injectable convenant pour être prélevée par une aiguille d'injection et être introduite dans ledit premier flacon pour constituer une seule unité posologique de (1R,5S,6S)-2-[(2-méthyl-1,2,3-thiadiazolium-4-yl)méthyl]thio-6-[(R)-1-hydroxyéthyl]-1-méthylcarbapénème-3-carboxylate.

9. Flacon stérile bouché de façon à empêcher une contamination et contenant du (1R,5S,6S)-2-[(2-méthyl-1,2,3-thiadiazolium-4-yl)méthyl]thio-6-[(R)-1-hydroxyéthyl]-1-méthylcarbapénème-3-carboxylate cristallin, ledit flacon comprenant une membrane dans laquelle peut pénétrer une aiguille d'injection.

**Revendications pour les Etats contractants suivants : ES, GR**

1. Procédé pour produire un (1R,5S,6S)-2-[(méthyl-1,2,3-thiadiazolium-4-yl)méthyl]thio-6-[(R)-1-hydroxyéthyl]-1-méthylcarbapénème-3-carboxylate cristallin représenté par la formule suivante :

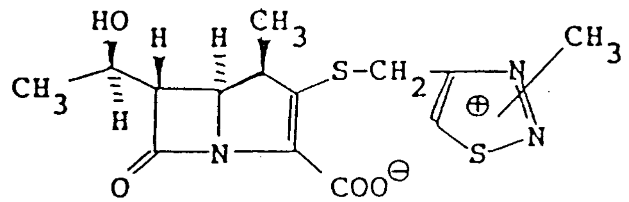

(I)

qui consiste à dissoudre du (1R,5S,6S)-2-[(méthyl-1,2,3-thiadiazolium-4-yl)méthyl]thio-6-[(R)-1-hydroxyéthyl]-1-méthylcarbapénème-3-carboxylate amorphe dans de l'eau à une concentration d'au moins 65 % et à faire précipiter des cristaux de la solution aqueuse.

2. Procédé selon la revendication 1 pour la production de (1R,5S,6S)-2-[(2-méthyl-1,2,3-thiadiazolium-4-yl)-méthyl]thio-6-[(R)-1-hydroxyéthyl]-1-méthylcarbapénème-3-carboxylate cristallin représenté par la formule :

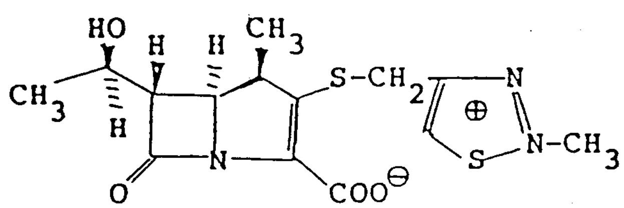

(I-1)